# EUROPEAN PATENT APPLICATION

(11) **EP 1 462 117 A1**
(43) Date of publication of application: **29.09.2004**
(21) Application number: 03290797.4
(22) Date of filing: 28.03.2003
(51) Int. Cl.: A61K 49/00, A61K 31/59

(54) **Method for in vivo detection of antipsychotic properties of dopamine D3 receptor ligands**

(71) Applicant: Institut National de la Santé et de la Recherche Médicale (INSERM), 75854 Paris Cedex 13 (FR)
(72) Inventor: Sokoloff, Pierre, 95130 le Plessis-Bouchard (FR); Leriche, Ludovic, 75012 Paris (FR)
(74) Representative: Bernasconi, Jean

(57) **Abstract**

The invention provides a method *in vivo* detecting anti-D₃ receptor activity of dopamine D₃ receptor ligands. The method of the invention makes use of a NMDA glutamate receptor antagonist to induce D₃-mediated behavioral abnormalities and assess reversing effects of candidate ligands. The invention further relates to the use of a dopamine D₃ receptor partial agonist or antagonist for the treatment of a substance-induced psychotic disorder or a dyskinesia associated with associated with dopamine agonist therapy.

## Description

Phencyclidine (PCP), a non-competitive antagonist at the N-methyl-D-aspartate (NMDA) subtype of glutamate receptor, produces schizophrenic-like symptoms in healthy volunteers (Luby et al., 1959; Jentsch et al., 1997) and precipitates psychosis in schizophrenic patients (Ital et al., 1967).

Psychotic symptoms induced by PCP, or the pharmacologically related compound ketamine, include both positive (hallucinations, delusions) and negative (formal thought disorder, social withdrawal) symptoms, as well as cognitive dysfunction (Cosgrove and Newell, 1991). Thus, administration of non-competitive NMDA receptor antagonist represents an impressive drug-induced model of schizophrenia (Javitt and Zukin, 1991; Snyder, 1988).

The ability of PCP to simulate schizophrenia has led to the proposal that the behavioral abnormalities evident in schizophrenic patients and PCP-exposed humans are caused by dysfunction of common neural substrates. The effect of PCP or its cognate molecules on brain dopamine systems has received particular attention (reviewed by Jentsch and Roth, 1999 and Svensson, 2000), since alteration in dopaminergic systems have been hypothesized in schizophrenia (Carlsson, 1988).

Convergent data suggest that locomotor effects of NMDA receptor antagonists are mediated via increased dopamine function, but the dopamine receptors involved have not been fully characterized.

The dopamine D₃ receptor (D₃R) is thought to be implicated in schizophrenia and treatment of this disorder (reviewed by Schwartz et al., 2000). D₃ receptors are localized in brain areas, which have been implicated in neural circuits believed to display defective functioning in schizophrenia and in PCP-treated animals (Carlsson, 1988; Jentsch and Roth, 1999; Svensson, 2000). Furthermore, a post-mortem study has shown a D₃ receptor overexpression in the brain of drug-free schizophrenic patients, whereas D₃ receptor expression was normal in subjects under antipsychotic drug treatment (Gurevich et al., 1997), suggesting that antipsychotic drugs normalize D₃ receptor expression. D₃ receptor overexpression in animals has been involved in sensitization to indirect dopamine agonists (Bordet et al., 1997; Le Foll et al., 2002), a clinical feature of schizophrenia (Janowsky and Davis, 1976). Finally, a meta-analysis indicates a genetic association of a polymorphism in the D₃ receptor gene with a small, but significant enhancement of the vulnerability to schizophrenia (Dubertret et al., 1998).

In animals, PCP or MK-801, another non competitive NMDA receptor antagonist (Wong et al., 1986), elicit behavioral abnormalities, including hyperactivity, disruption of sensorimotor gating and social deficit, that are circumvented by antipsychotic drugs, particularly of the atypical type (Jentsch and Roth, 1999).

The inventors have now demonstrated that the corrective effects of antipsychotic drugs on hyperactivity behavioral abnormalities produced by NMDA antagonists at low, non doses, involve D₃ receptor blockade.

The inventors more particularly administered wild-type or D₃ receptor knockout mice with (NMDA)/glutamate receptor antagonists, which generate schizophrenic-like symptoms in humans and behavioral abnormalities in animals mimicking abnormalities found in schizophrenia, such as hyperactivity. They then assessed the behaviors of the mice so treated.

The effects of antipsychotic drugs have further been compared with those produced by D₃ receptor-preferring antagonist or partial D₃ receptor agonists in wild-type and D₃ receptor knockout mice models.

The inventors thus showed that the psychotic behavior induced by NMDA antagonists is dependent upon D₃ receptor stimulation. The mammal model described herein constitutes the first simple response to assess the in vivo activity of D₃ receptor-selective drugs. The present invention provides a test for detection of antipsychotic properties of drugs selectively acting on D₃ receptors.

Interestingly, psychotic symptoms induced by NMDA antagonists such as PCP are more similar to schizophrenia when the drug is taken chronically. In particular, negative symptoms of the disease (formal thought disorder, social withdrawal) symptoms, as well as cognitive dysfunction (Cosgrove and Newell, 1991) appear after repeated consumption of the drug.

Accordingly, the invention also provides an animal model of chronic NMDA receptor blockade which produces behavioral disturbances mimicking symptoms of schizophrenia, (*i.e.* spontaneous hyperactivity, hypersensitivity to psychostimulants and social interaction deficit)

### Definitions

As used herein, the term *"NMDA"* denotes the N-methyl-D-aspartate (NMDA) subtype of glutamate receptor.

A *"NMDA receptor antagonist"* or *"NMDA glutamate receptor antagonist"* refers to a compound which inhibits, partially or totally, function of NMDA receptor. Examples of NMDA receptor antagonists include for instance phencyclidine (1-(1-phencyclohexyl)-piperidine, PCP), 1-[1-(2-thienyl)-cyclohexyl]piperidine (TCP), ketamine (2-(2-(chlorophenyl)-2-(methylamino)cyclohexanone), dizocilpine [(+)-5-methyl-10,11-dihydro-5H-dibenzo[a,d]cyclohepten-5,10-imine maleate, (+)-MK-801], dextrorphan ((+)-3-hydroxy-*N*-methylmorphinan), N-allyl-normetazocine (SKF 10,047), cis-4-phosphonomethyl-2-piperidine carboxylic acid (CGS 19755), 2R,4R,5S 2-amino-4,5-(1,2-cyclohexyl)-7-phosphonoheptanoic acid (NCP 17742), (±)-2-carboxypiperazine-4-yl-propyl-1-phosphonic acid [(±)-CPP], amantadine (tricyclo[3.3.1.1^{3,7}]decan-1-amine) and memantine (3,5-dimethyltricyclo[3.3.1.1^{3,7}]decan-1-amine).

As used herein, the term *"dopamine D*_{*3*} *receptor", "D*_{*3*} *receptor",* or "*D*_{*3*}" denotes a subtype of dopamine receptor principally expressed in the limbic system (Sokoloff et al., 1990). D₃ receptor has been further described in the international patent application WO 91/15513.

The term *"D*_{*3*} *receptor ligand",* as used herein, refers to a molecule that has the capacity to bind the dopamine D₃ receptor. Upon binding, a ligand may or may not modify or interfere with the activity of the D₃ receptor. D₃ receptor ligands include D₃ receptor partial agonists and antagonists. D₃ receptor ligands further include *"D*_{*3*} *receptor selective ligands",* i.e. ligands that are selective for D3 receptor and that do not bind to other receptors, such as the dopamine D₂ receptor or the α1-adrenergic receptor.

The term *"D*_{*3*} *receptor partial agonist"* is intended for a compound that forms a complex with the D₃ receptor and acts as a mixed agonist-antagonist. *In vitro,* a partial agonist of the D₃ receptor produces in a cell expressing the D₃ receptor an active response, of which the maximal intensity is lower than that produced by dopamine or its full agonist, for instance quinpirole (*trans*-(-)-4aR-4,4a, 5,6,7,8,8a,9-octahydro-5-propyl-1H(or 2H)-pyrazolo[3, 4-g]quinoline). A D₃ receptor partial agonist is also able to partially inhibit the response produced by dopamine or its full agonists. *In vivo,* a D₃ receptor partial agonist produces dopaminomimetic responses, especially when dopamine is depleted such as in 6-hydroxydopamine-lesioned rats or in 1-methyl-4-phenyl-1,2,3,6-tetrahydropyridine (MPTP)-treated monkeys. Additionally, *in vivo* a D3 partial agonist may act as an antagonist, especially when the D₃ receptor is subject to a sustained stimulation by dopamine. Examples of D₃ receptor partial agonists include BP 897 (1-(4-(2-Naphthoylamino)butyl)-4-(2-methoxyphenyl)-1A-piperazine, Pilla et al., 1999) and aripiprazole (7-[4-[4-(2, 3 dichlorophenyl)-1-piperazinyl]butoxy]-3, 4-dihydro-2(1*H*)-quinoline, Lawler et al., 1999).

A *"D*_{*3*} *receptor antagonist"* denotes a molecule that forms a complex with D₃ receptor and that is capable of inhibiting an active response triggered by dopamine or its agonists from a cell expressing the D₃ receptor. Examples of D₃ receptor antagonists include nafadotride (N[(n-butyl-2-pyrrolidinyl)methyl]-1-methoxy-4-cyano naphtalene-2-carboxamide, Sautel et al. 1995), ST 198 ((E)-N-(4-[1,2,3,4-tetrahydroisoquinolin-2-yl]-butyl)-3-phenylacrylamide), SB-277701A (*trans-N-*[4-[2-(6-cyano-1,2,3,4-tetrahydroisoquinolin-2-yl)ethyl]cyclohexyl]-4-quinolinecarboxamide, Reavill et al., 2000), S33084 (3a*R*,9b*S*)-*N*-[4-(8-cyano-1,3a,4,9b-tetrahydro-3H-benzopyrano[3,4-c]pyrrole-2-yl)butyl]'-phenyl) benzamides, Millan et al., 2000).

By *"psychotic behavior"* is meant a behavior associated with psychosis. Psychosis is an impairment of mental functioning to the extent that it interferes with an individual's ability to meet the ordinary demands of life. According to the American Psychiatric Association, psychotic means grossly impaired, in terms of reality testing. Such testing would define gross impairment as existing when individuals incorrectly evaluate the accuracy of their perceptions and thoughts and make incorrect inferences about external reality, even in the face of contrary evidence. The term psychotic is also appropriate when behavior is so disorganized that it is reasonable to infer that results of reality testing would indicate an individual as grossly disturbed, for instance, by the existence of markedly incoherent speech without apparent awareness by the person that the speech is not understandable or by the agitated, inattentive and disoriented behavior observed in the phencyclidine psychotic disorder (Diagnostic and Statistical Manual of Mental Disorders, Ed. 4, American Psychiatric Association, Washington, D.C. 1994; Kaplan & Sadock's Comprehensive Textbook of Psychiatry, Seventh Edition, Volume I, Lippincott Williams & Wilkins: Philadelphia, pp. 825, 2000). Psychotic disorders include, and are not limited to, schizophrenia, schizophreniform disorder, schizoaffective disorder, delusional disorder, brief psychotic disorder, shared psychotic disorder, psychotic disorder due to a general medical condition, or psychotic disorder not otherwise specified (Diagnostic and Statistical Manual of Mental Disorders, Ed. 4, American Psychiatric Association, Washington, D.C. 1994). In particular, in the context of the invention psychotic disorders include *"substance-induced psychotic disorder"* and *"psychotic-like behavior".*

By *"substance-induced psychotic disorder"* is meant an abnormal behavior resembling psychotic disorder in humans, resulting from consumption or administration of NMDA receptor antagonists. Conditions of this type includes, but is not limited to, disorders resulting from consumption of drugs of abuse, such as PCP ("angel dust") and ketamine, administration of anesthetics such as ketamine, administration of neuroprotective agents, such as memantine in the treatment of Alzheimer disease and vascular dementia, and administration of amantadine in the treatment of Parkinson's disease. Substance-induced psychotic behaviors include psychotic-like behaviors.

By *"psychotic-like behavior"* is meant an abnormal behavior in a non human mammal that is elicited by a drug producing a substance-induced psychotic behavior in humans, for instance phencyclidine or other NMDA receptor antagonists. Examples of psychotic-like behaviors include locomotor abnormalities and deficits in working or spatial memory, deficit in social interactions, deficit in sensorimotor gating and deficit in latent inhibition.

The term *"candidate molecule"* is intended for a molecule of known or unknown structure, or a mixture of molecules, or an extract of a natural product. Preferably, a candidate molecule is a molecule that has already been identified as a D₃ receptor ligand, still preferably as a D₃ receptor selective ligand. Such identification may be achieved by any appropriate characterization method, such as receptor binding assay. Methods for assessing binding to a receptor, in particular D₃, are well-known by the one skilled in the art. For instance reference may be made to the methods described in Pilla et al., 1999, and Pilon et al., 1994, Sautel et al., 1995.

The term *"a dopamine D*_{*3*} *ligand having anti-D*_{*3*} *receptor activity in vivo"* is intended for a ligand that can inhibit an active response triggered by dopamine or its agonists *in vivo.* In the context of the invention ligands identified as having anti-D₃ receptor activity *in vivo* include in particular D₃ receptor antagonists or partial agonists. Examples of anti-D₃ receptor activity include for instance antipsychotic activity, anti-dyskinesic activity, or reduction of drug craving and vulnerability to relapse in drug-abusers (Pilla et al., 1999).

The term *"antipsychotic"* or *"antipsychotic activity"* is used for a molecule that allows reduction of a psychotic behavior, including a substance-induced psychotic behavior and a psychotic-like behavior, when administered to a psychotic patient.

The term "anti-dyskinesic activity" is meant for a molecule that allows treatment of a dyskinesia, such as a dyskinesia associated with dopamine agonist therapy, as defined below,

As used herein the term *"mammal'* denotes a mammal, including a human or a non-human mammal, including rodents (*e.g.* a rat, a mouse or a rabbit), monkeys, cattle, sheep, feline (*e.g.* a cat), canine (*e.g.* a dog), horses, goats...

In the context of the invention, the term *"treating"* or *"treatment"*, as used herein, means reversing, alleviating, inhibiting the progress of, or preventing the disorder or condition to which such term applies, or one or more symptoms of such disorder or condition.

*"Pharmaceutically"* or *"pharmaceutically acceptable"* refer to molecular entities and compositions that do not produce an adverse, allergic or other untoward reaction when administered to an animal, or a human, as appropriate.

As used herein, *"pharmaceutically acceptable carrier"* includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

### Screening methods

The present invention provides a method for identifying a dopamine D₃ receptor ligand having anti-dopamine D₃ receptor activity *in vivo,* said method comprising a) administering a mammal with (i) a candidate molecule and (ii) a NMDA glutamate receptor antagonist in a amount suitable for inducing dopamine D₃ receptor-dependent psychotic behavior in said mammal, and

b) determining the capacity of the candidate molecule to prevent or inhibit the psychotic behavior induced in the mammal, wherein a reduced psychotic behavior or non-appearance thereof is indicative of a dopamine D₃ receptor ligand having anti-dopamine D₃ receptor activity *in vivo.*

According to an embodiment, the candidate molecule is administered prior to the NMDA glutamate receptor antagonist, and the psychotic behavior of the mammal is assessed before and after each administration, and compared with the behavior of a control mammal to which a control vehicle has been administered in lieu of the candidate molecule. A reduced psychotic behavior, or non-appearance thereof, with comparison to the behavior of the control mammal is indicative of a dopamine D₃ receptor ligand having anti-dopamine D₃ receptor activity *in vivo.*

Preferably, the *in vivo* anti-dopamine D₃ receptor activity of the dopamine D₃ receptor ligand is an anti-psychotic activity.

In particular the psychotic behavior induced in a mammal is a substance-induced psychotic disorder or a psychotic-like disorder.

This may be achieved by administrating a mammal with the candidate molecule or a control vehicle and measuring said mammal's spontaneous activity for a given period of time, for instance for the first 30 minutes post-administration. Afterwards, a NMDA glutamate receptor antagonist may be administered, at an appropriate dosage, to a mammal that received either the control vehicle *("control mammal")* or the candidate molecule ("*test mammal").* Activity under the influence of the NMDA antagonist may be measured for time sufficient to allow development of a psychotic behavior in a control mammal, for instance 60 minutes with (+)-MK-801, so as to compare psychotic behavior in the test and control mammals. Non-appearance of psychotic behavior in the test mammal, or reduced development of the psychotic behavior compared with the control mammal, indicates that the candidate molecule is a D₃ receptor ligand having antipsychotic activity *in vivo.*

According to another embodiment, the candidate molecule is administered after the NMDA glutamate receptor antagonist, and the psychotic behavior of the mammal is assessed before and after each administration, and compared with the behavior of a control mammal to which a control vehicle has been administered in lieu of the candidate molecule. A reduced psychotic behavior with comparison to the behavior of the control mammal is indicative of a dopamine D₃ receptor ligand having antipsychotic activity *in vivo.*

This may be achieved by administrating to a mammal the NMDA receptor antagonist and measuring activity under the influence of said antagonist for a time sufficient to evaluate the psychotic behavior of said mammal. The control vehicle (control mammal) or the candidate molecule (test mammal) is further administered to a mammal that received the NMDA receptor antagonist. The activity of the test and control mammals may be measured as long as a psychotic behavior is observed in the control mammal. Reduced psychotic behavior in the test mammal compared with the control mammal indicates that the candidate molecule is a dopamine D₃ receptor ligand having antipsychotic activity *in vivo.*

According to this latter embodiment, said NMDA glutamate receptor antagonist may be administered continuously, *i.e*. by infusion, or chronically to the mammal for a time sufficient to allow development of a psychotic behavior in said mammal, prior to administrating the candidate molecule. For instance, the NMDA glutamate receptor antagonist may be infused until development of a psychotic behavior, for instance for at least 7 days with dizocilpine, and prolonged for a time sufficient to enable assessment of the candidate molecule activity of said psychotic behavior, for instance for up to 10 days with dizocilpine. It is generally observed that the psychotic behavior maintains for a few days after cessation of NMDA glutamate receptor antagonist infusion. The duration and cessation time of infusion can be readily adapted by the skilled in the art depending on the type and dosage of the NMDA receptor antagonist and on the mammalian species used.

Preferably, said NMDA glutamate receptor antagonist is selected from the group consisting of phencyclidine, TCP, ketamine, +(-)MK-801, dextrorphan, SKF 10,047, CGS 19755, NCP 17742, and (±)-CPP.

Preferably, said psychotic behaviors, and in particular substance-induced psychotic behaviors, include but are not limited to alterations of perception, impaired performance on tests of vigilance, attention and verbal fluency, impaired performance on the Wisconsin Card Sorting Test, symptoms similar to dissociative states, alteration of preattentive auditory event-related potential, impaired recall and recognition memories (Krystal et al., 1994). Psychotic behaviors can also be assessed by using psychiatric scales such as the Brief Psychiatric Rating Scale, Scale for the Assessment of positive Symptoms and Scale for Assessment of Negative Symptoms.

Also preferably, said psychotic behaviors, such as psychotic-like behaviors, include spontaneous locomotor hyperactivity, enhanced locomotor responses to psychostimulants such as amphetamine, methamphetamine or cocaine, deficit in social interactions (measured for instance in the resident-intruder test), deficit in sensorimotor gating (measured for instance as a disruption of prepulse inhibition) or deficit in working or spatial memory (measured for instance with the Y-maze and Morris water maze, respectively).

The mammal is administered with the candidate molecule or NMDA glutamate receptor antagonist in any appropriate form.

More particularly, the candidate molecule or NMDA glutamate receptor antagonist may be administered orally, parenterally (including subcutaneous, intravenous, intramuscular or intrasternal injection or infusion techniques), by inhalation spray, or rectally, in dosage unit formulations containing conventional non-toxic pharmaceutically-acceptable carriers, adjuvants and vehicles.

The candidate molecule or NMDA glutamate receptor antagonist may thus be in the form of orally-administrable suspensions or tablets; nasal sprays; sterile injectable preparations, for example, as sterile injectable aqueous or oleagenous suspensions or suppositories.

A candidate molecule may be administered according to dosage established in the art whenever specific blockade of the D₃ receptor is required.

### Psychotic behavior induction

A NMDA receptor antagonist may be administered in any form and according to dosage suitable for inducing D₃-dependent psychotic behavior in a mammal. Examples of preferred dosage are indicated below, depending on whether an acute or continuous/chronic NMDA receptor antagonist administration is implemented (Geter-Douglas and Witkin, 1997; Irifune et al., 1995) :

| Compound | Dosage | |
|---|---|---|
| | Acute (i.p.) | Continuous (infusion) |
| Phencyclidine | 0.1-3 mg/kg | 0.05-0.3 mg/kg/h |
| TCP | 0.05-1 mg/kg | 0.04-0.2 mg/kg/h |
| Ketamine | 0.1-10 mg/kg | 0.01-5 mg/kg/h |
| Dizocilpine ((+) MK-801) | 0.05-0.2 mg/kg | 0.005-0.04 mg/kg/h |
| (-)MK-801 | 0.05-1 mg/kg | 0.04-0.2 mg/kg/h |
| Dextrorphan | 1-30 mg/kg | 1-6 mg/kg/h |
| SKF 10,047 | 0.5-15 mg/kg | 0.5-3 mg/kg/h |
| CGS 19755 | 0.5-20 mg/kg | 0.6-4 mg/kg/h |
| NPC 17742 | 1-30 mg/kg | 1-6 mg/kg/h |
| (±)-CPP | 2-70 mg/kg | 2-12 mg/kg/h |

Based on the present specification, the one skilled in the art can readily determine the NMDA receptor antagonist dosage appropriate for inducing D₃-dependent psychotic behavior. As shown in Example 1, dependence on D₃ receptor may be confirmed by comparing the inhibiting effect of a known antipsychotic D₃-ligand, such as BP 897, on the psychotic behavior induced in wild-type mice (D₃R^{+/+}) or D₃ receptor-deficient mice (D₃R^{-/-}).

### Therapeutic methods

According to the ability of D₃ receptor partial agonists and antagonists to reduce psychotic behaviors, such as in particular substance-induced psychotic behaviors, it is proposed a method for reducing or preventing psychotic behaviors elicited by medications containing NMDA receptor antagonists. These medications include anesthetics (for instance ketamine), medications used in the treatment of Alzheimer disease and vascular dementia (for instance memantine) and medications used in the treatment of Parkinson's disease (amantadine).

The invention thus provides a method for treating a substance-induced psychotic disorder that comprises administering a patient in need thereof with a dopamine D₃ ligand having anti-D₃ receptor activity *in vivo.*

Accordingly, the invention also relates to the use of a dopamine D₃ receptor ligand having anti-D₃ receptor activity *in vivo* for the manufacture of a medicament intended for the treatment of a substance-induced psychotic disorder in a patient in need thereof.

Identification of dopamine D₃ ligand having anti-D₃ receptor activity *in vivo*, and in particular having antipsychotic activity *in* two, can be readily achieved by the one skilled in the art in view of the screening methods described above and the examples provided herein.

Preferably, said dopamine D₃ receptor ligand is a partial agonist or antagonist of the D₃ receptor. A dopamine D₃ receptor partial agonist or antagonist may be selected from the group consisting of BP 897, aripiprazole, nafadotride, ST 198, SB-277701A and S3 3084. Still preferably said dopamine ligand is the partial agonist BP 897.

Also preferably said substance induced psychotic disorder is associated with the administration of a NMDA antagonist, and in particular with administration of an anesthetic or a medication for the treatment of disorders such as Alzheimer disease, vascular dementia, or Parkinson's disease. Additionally, said substance-induced psychotic behavior may be associated with abuse of psychoactive drugs as ketamine or phencyclidine.

According to this embodiment, the term *"patient"* or *"patient in need thereof"*, is intended for a human or non-human mammal affected or likely to be affected with a substance-induced psychotic behavior, such a human or non-human mammal treated with a NMDA receptor antagonist as described above.

According to another aspect, it is provided a method for reducing or eliminating the involuntary movements, *i.e*. dyskinesia, induced by dopamine agonist treatments of disorders such as Parkinson's disease (PD).

Actually, therapeutic interventions on neurotransmission could benefit from the use of a partial receptor agonist acting as a stabilizing agent, which could limit neurotransmitter fluctuations in either direction, *i.e.* maintain substantial and blunt excessive receptor stimulations.

This concept has been illustrated by the inventors by using a primate model of Parkinson's disease (PD), a condition caused by the lack of the brain neurotransmitter dopamine. Substitution treatment of PD by the dopamine precursor levodopa initially reduces motor symptoms, but on the long term eventually induces, in a majority of patients, debilitating and pharmacoresistant involuntary movements, *i.e*. dyskinesia, presumably resulting from an excessive dopamine response (see Bezard et al., 2001 for a recent review)

Monkeys rendered parkinsonian with 1-methyl-4-phenyl-1,2,3,6-tetrahydropyridine (MPTP) have decreased dopamine D₃ receptor levels, but dyskinesia induced by long-term administration of levodopa is associated with an overexpression of this receptor. The inventors have demonstrated that administration of a D₃ receptor-selective partial agonist strongly attenuates levodopa-induced dyskinesia, leaving unaffected the therapeutic effect of levodopa. On the contrary, attenuation of dyskinesia by D₃ receptor antagonists is accompanied by the reappearance of PD-like symptoms.

In view of D₃ receptor involvement in both dyskinesia and therapeutic action of levodopa, a method of therapeutic treatment is proposed that makes use of the anti-dyskinesic activity of partial agonists of D₃ receptor to normalize exacerbated D₃ receptor function and correct side-effects of levodopa in PD patients.

The invention thus further provides a method for treating dyskinesia associated with dopamine agonist therapy, said method comprising administering a partial agonist of the dopamine D₃ receptor to a patient in need thereof.

The invention also relates to the use of a partial agonist of the dopamine D₃ receptor for the manufacture of a medicament intended for the treating dyskinesia associated with dopamine agonist therapy in a patient in need thereof.

The term *"treating"* or *"treatment",* as used herein, means reversing, alleviating, inhibiting the progress of, or preventing the disorder or condition to which such term applies, or one or more symptoms of such disorder or condition.

By the term *"patient", "patient in need thereof"*or *"patient in need of such treatment"* is meant any human or non-human mammal affected or likely to be affected with dyskinesia, such as for instance a human or non-human mammal with Parkinson's disease (PD) treated with levopoda.

The term *"dyskinesia",* as used herein means any abnormal or uncontrollable movement including, but not limited to, chorea, tremor, ballism, dystonia, athetosis, myoclonus and tic. Dyskinesias often result from treatment of the physical symptoms of Parkinson's disease. Parkinson's disease is characterized by tremor, rigidity, bradykinesia and postural instability. Such involuntary physical movements may be reduced by therapies which increase dopamine receptor stimulation.

In the context of the invention, the term *"dyskinesia associated with dopamine agonist therapy",* as used herein, unless otherwise indicated, means any dyskinesia which accompanies, or follows in the course of, dopamine agonist therapy, or which is caused by, related to, or exacerbated by dopamine agonist therapy, wherein dyskinesia and dopamine agonist therapy are as defined above.

*"Dopamine agonist therapy"* is intended for any therapy that increases dopamine receptor stimulation, including therapies that directly stimulate dopamine receptors and therapies that increase the levels of dopamine. Dopamine agonist therapy, as referred to in the present invention, is used in the treatment of a central nervous system disorder such as Parkinson's disease. Dopamine agonist therapies may include administration of agents such as: L-dopa (also named levodopa), dopamine D1 or D2 receptor agonists; dopamine/beta-adrenergic receptor agonists, dopamine/5-HT uptake inhibitor/5-HT-1A agonists, alpha2-adrenergic antagonist/dopamine agonists, or dopamine precursors. Preferably said dopamine agonist therapy is a treatment of PD comprising administration of levopoda, or a combination of levodopa with any dopamine agonist.

Advantageously, *in vivo* anti-D₃ receptor activity of said partial agonist of the dopamine D₃ receptor may be assessed by a screening method according to the invention. Preferably said partial agonist of the dopamine D₃ receptor is selected from the group consisting of BP 897 and any drug possessing D₃ receptor partial agonist property, for instance aripiprazole.

According to the method or use described above, the partial agonist of the dopamine D₃ receptor may be in any appropriate form.

In the above therapeutic methods or applications, the dopamine D₃ receptor ligand, and in particular D₃ receptor partial agonist or antagonist, may be administered orally, parenterally (including subcutaneous injections, intravenous, intramuscular, intrasternal injection or infusion techniques), by inhalation spray, or rectally, in dosage unit formulations containing conventional non-toxic pharmaceutically-acceptable carriers, adjuvants and vehicles.

Such pharmaceutical compositions containing a ligand of the dopamine D₃ receptor alone or in combination with another D₃ ligand or another active ingredient may thus be in the form of orally-administrable suspensions or tablets; nasal sprays; sterile injectable preparations, for example, as sterile injectable aqueous or oleagenous suspensions or suppositories.

When administered orally as a suspension, these compositions are prepared according to techniques well-known in the art of pharmaceutical formulation and may contain microcrystalline cellulose for imparting bulk, alginic acid or sodium alginate as a suspending agent, methylcellulose as a viscosity enhancer, and sweeteners/flavoring agents known in the art. As immediate release tablets, these compositions may contain microcrystalline cellulose, dicalcium phosphate, starch, magnesium stearate and lactose and/or other excipients, binders, extenders, disintegrants, diluents and lubricants known in the art.

When administered by nasal aerosol or inhalation, these compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art.

The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

When rectally administered in the form of suppositories, these compositions may be prepared by mixing the drug with a suitable non-irritating excipient, such as cocoa butter, synthetic glyceride esters or polyethylene glycols, which are solid at ordinary temperatures, but liquidify and/or dissolve in the rectal cavity to release the drug.

A D₃ ligand, such as a partial agonist or antagonist of the D₃ receptor may be administered in any of the foregoing compositions and according to dosage regimens established in the art whenever specific blockade of the human D₃ receptor is required.

The daily dosage of the products may be varied over a wide range from 0.01 to 1,000 mg per adult human per day. For oral administration, the compositions are preferably provided in the form of tablets containing 0.01, 0.05, 0.1, 0.5, 1.0, 2.5, 5.0, 10.0, 15.0, 25.0, 50.0, 100, 250 and 500 milligrams of the active ingredient for the symptomatic adjustment of the dosage to the patient to be treated. A medicament typically contains from about 0.01 mg to about 500 mg of the active ingredient, preferably, from about 1 mg to about 100 mg of active ingredient. An effective amount of the drug is ordinarily supplied at a dosage level of from about 0.0002 mg/kg to about 20 mg/kg of body weight per day. Preferably, the range is from about 0.001 to 10 mg/kg of body weight per day, and especially from about 0.001 mg/kg to 7 mg/kg of body weight per day. The compounds may be administered on a regimen of 1 to 4 times per day. It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

The invention will be further illustrated by the following figures and examples.

### FIGURES

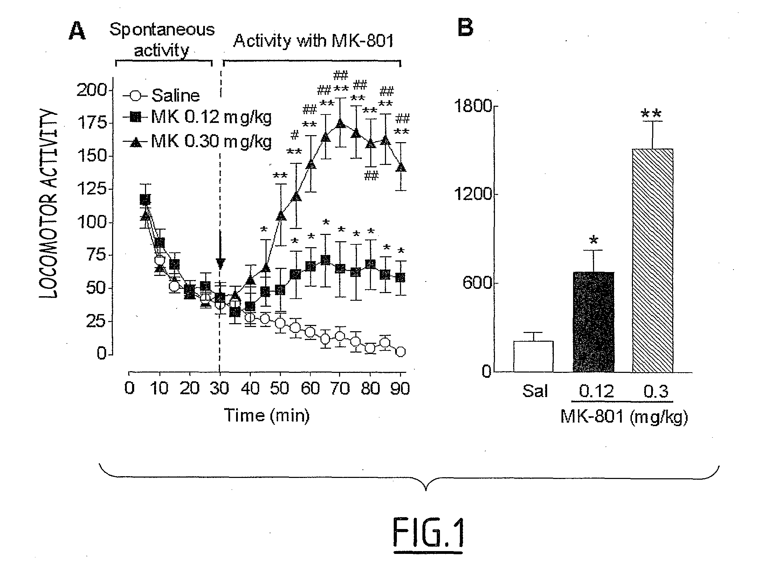

Figure 1 illustrates hyperlocomotion induced by MK-801 in the mouse. **A,** After a 30 min habituation period (spontaneous activity), MK-801 (0.12 or 0.3 mg/kg i.p.) or saline was administered (arrow), and the locomotor activity (activity with MK-801) was recorded during 60 min. Results are means of counts ± SEM (*n* = 10-12). * P < 0.05, ** P < 0.01 vs. saline-treated mice. # P < 0.05, ## P < 0.01 vs. MK-801 0.12 mg/kg. **B**, locomotor activity summed up for 60 min after administration of saline (Sal) or MK-801 at the indicated dosage. * P <0.01; ** P < 0.001 vs. Sal.

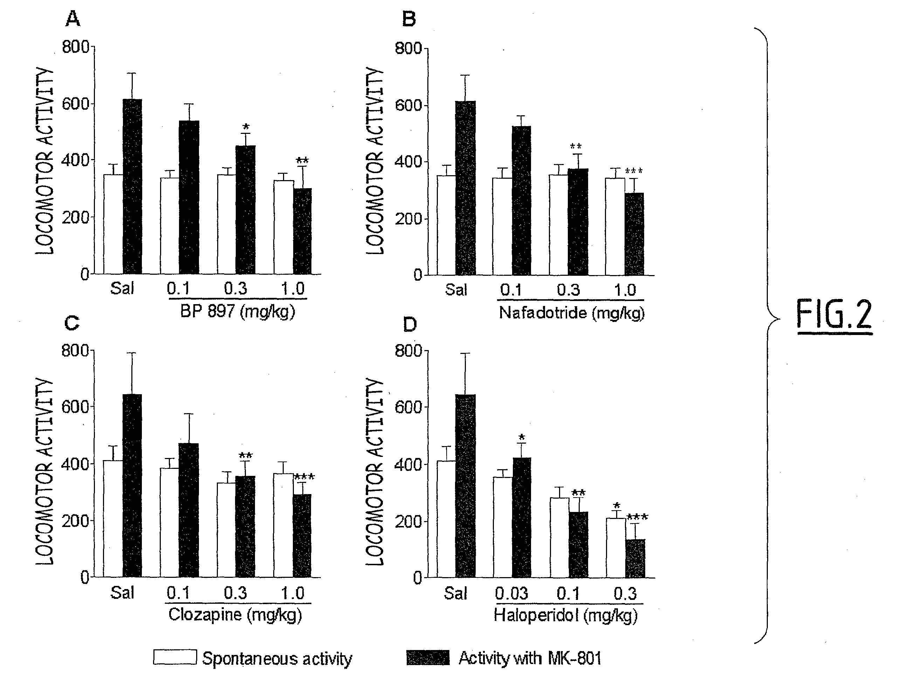

Figure 2 describes the effect of BP 897 **(A),** nafadotride **(B),** clozapine **(C),** or haloperidol **(D)** on spontaneous and MK-801-induced locomotor activity. Animals received an injection of the compounds at the indicated dosage or saline (Sal) and, starting 5 min after the injection, their locomotor activity was measured during 30 min (spontaneous activity, *white bars).* Then, MK-801 (0.12 m/kg) was administered and the locomotor activity recorded during 60 min (activity with MK-801, *black bars).* Data are means ± S.E.M. (*n* = 10-12). * P < 0.05, ** P < 0.01, *** P < 0.005 vs. respective Sal.

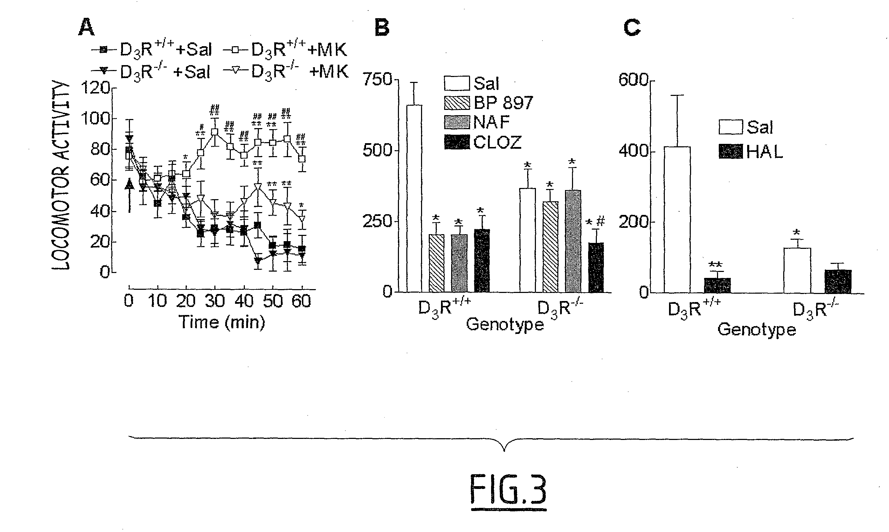

Figure 3. illustrates the inhibition of MK-801-induced activity by D₃ receptor antagonists and antipsychotic drug in D₃R^{+/+} or D₃R^{-/-}. **A** Following a 30-min period of habituation, saline (Sal) or MK-801 (MK, 0.12 mg/kg) was injected (*arrow*) to wild-type (D₃R^{+/+}) or D₃ receptor-deficient mice (D₃R^{-/-}) and locomotor activity was recorded during 60 min. Data represent means ± SEM (*n* = 10-12). * P < 0.05, ** P < 0.01, vs. respective saline-treated mice, # P < 0.05, ## P < 0.01 vs. MK-801-treated D₃R^{-/-} mice. **B,** Saline (Sal), BP 897 (1 mg/kg), nafadotride (NAF, 1 mg/kg) or clozapine (CLOZ, 1 mg/kg) were injected 5 min before placement in the actimeter and the locomotor activity recorded for 30 min (spontaneous activity). MK-801 (0.12 mg/kg) was then administered and locomotor activity recorded for 60 min. * P < 0.001 vs. saline-treated D₃R^{+/+} mice, # P < 0.05 vs. saline-treated D₃R^{-/-} mice. Data represent means ± SEM (*n* = 20-24) of locomotor counts recorded when MK-801 was present. **C**, the same experiment as in B was performed with haloperidol (HAL, 0.05 mg/kg). * P < 0.01, ** P < 0.001 vs. saline-treated D₃R^{+/+} mice (*n* = 8).

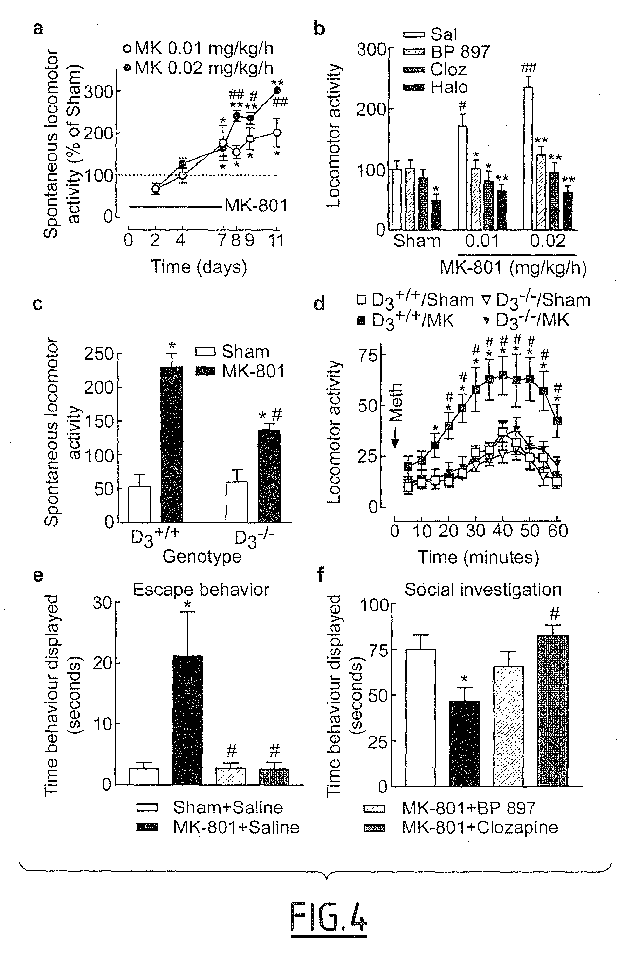

Figure 4 shows behavioral abnormalities induced by a continuous infusion of MK-801, and their correction either by acute treatments with clozapine or haloperidol, two antipsychotic drugs, or with BP 897, a highly D₃ receptor-selective partial agonist, or in D₃ receptor knockout mice. **a,** Spontaneous and long-lasting locomotor hyperactivity induced by a 7-day continuous systemic infusion with MK-801 at 0.01 and 0.02 mg/kg/h. Results are means ± S.E.M (*n* = 8) expressed as percentage of sham-operated animals (Sham). * *P* < 0.05, ** *P* < 0.01 vs. Sham. ^{#} P < 0.05 and ^{##} P < 0.01 vs. MK 0.01 mg/kg/h. **b,** Inhibition of MK-801-induced spontaneous hyperactivity by BP 897 (1 mg/kg), clozapine (Cloz, 1 mg/kg) or haloperidol (Halo, 0.3 mg/kg). Results are means ± S.E.M. (*n* = 12). * P < 0.05 and ** P<0.01 vs. respective saline-treated animals (Sal). ^{#} P < 0.05 and ^{##} P < 0.01 vs. Sham + Sal. **c,** Attenuation of spontaneous hyperactivity induced by MK-801 (0.02 mg/kg/h for 7 days) in D₃ receptor knockout (D₃^{-/-}), compared to wild-type (D₃^{+/+}) mice. Results are means ± S.E.M. (*n* = 6-7). *P < 0.05 vs. respective Sham. ^{#}P < 0.05 vs. D₃^{+/+} mice treated with MK-801. **d,** MK-801 (MK, 0.02 mg/kg/h during 7 days) continuous treatment induced a hypersensitivity to methamphetamine (Meth, 1 mg/kg i.p., time of injection indicated by an arrow), in D₃^{+/+} mice, which was abolished in D₃^{-/-} mice. Results are means ± S.E.M. (*n* = 9-10). * P < 0.05 vs. D₃^{+/+}/Sham. ^{#} P < 0.05 vs. D₃^{-/-}/MK. **e, f,** Social withdrawal in MK-801-treated mice (0.02 mg/kg/h for 7 days), quantified with the resident-intruder behavioral assay over a 5-min period of time, was corrected by clozapine (1 mg/kg) or BP 897 (1 mg/kg) administered acutely. **e**, Time spent by the resident male actively avoiding social interaction (escape behavior). **f**, Time spent by the resident male actively pursuing social investigation of the intruder mouse. Results are means ± S.E.M. (*n* = 10). * P < 0.05 vs. Sham + Saline, ^{#} P < 0.05 vs. MK-801 + Saline.

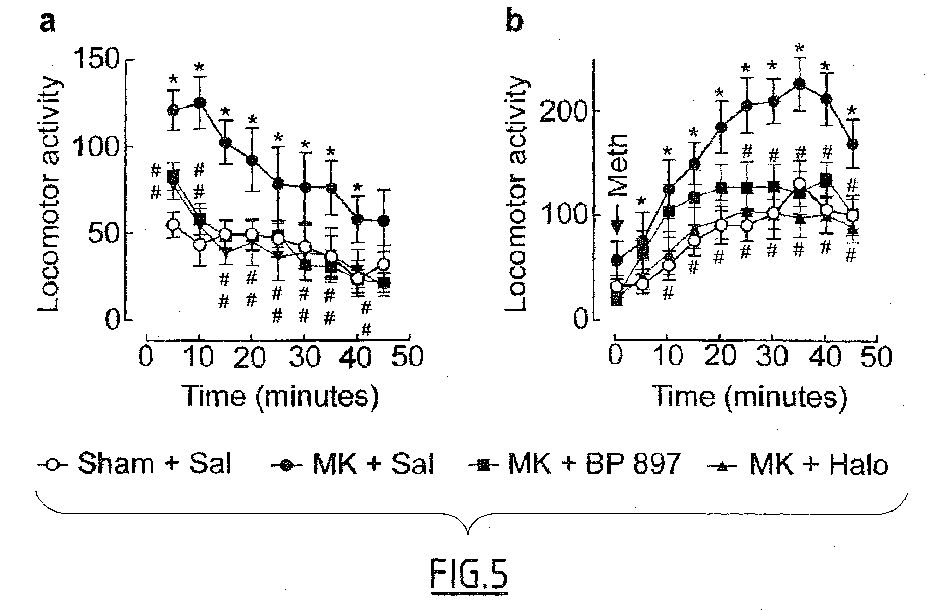

Figure 5 illustrates that a subchronic treatment with BP 897 or haloperidol, initiated at the emergence of the troubles (6 days after the beginning of MK-801 continuous delivery), corrected MK-801-induced behavioral abnormalities. **a**, The spontaneous locomotor hyperactivity after MK-801 treatment (0.02 mg/kg/h during 7 days, MK + Sal) was abolished by a subchronic treatment (twice a day for 4.5 days) with BP 897 (1 mg/kg, MK + BP 897) or haloperidol (0.3 mg/kg, MK + Halo). **b**, Hypersensitivity to methamphetamine (1 mg/kg, Meth, time of injection indicated by an arrow) developing after the MK-801 treatment was abolished by the same treatments. Results are means ± S.E.M. of values from 12 animals. * P < 0.05 vs. sham-operated, saline-treated animals (Sham + Sal). ^{#} P < 0.05 vs. MK + Sal.

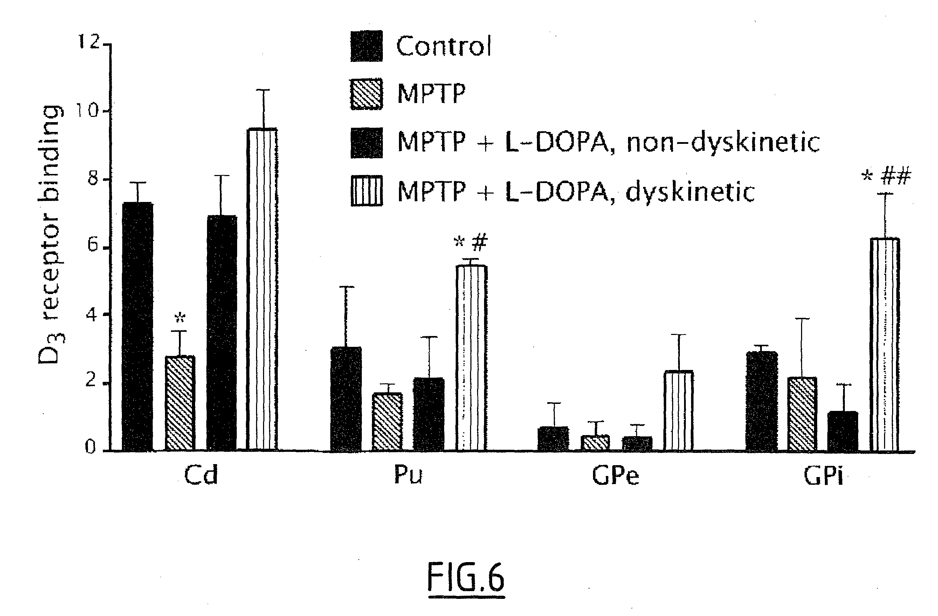

Figure 6 represents the results of D₃ receptor expression assessment in dyskinesia and shows that dyskinesia is accompanied by D₃ receptor overexpression. Quantitative analysis of typical receptor autoradiograms obtained with [¹²⁵I]7-OH-PIPAT, a selective D₃ receptor radioligand, in the brain from typical a normal monkey, untreated MPTP-intoxicated monkeys (MPTP), and non dyskinetic and dyskinetic MPTP-intoxicated monkeys treated by levodopa (L-DOPA). Results are mean ± S.E.M. of values in µCi/mg. Treatments had significant effects in caudate nucleus (Cd, F_{3,11} = 6.93, *P* = 0.007 by analysis of variance), putamen (Pu, F_{3,11} = 4.51, *P* = 0.026) and internal part of globus pallidus (GPi, F_{3,11} = 4.47, *P* = 0.028), but not in external part of globus pallidus (GPe, F_{3,11} = 1.70, *P* = 0.22). *, *P* < 0.05 vs. control; §, *P* <0.05;, §§, *P* < 0.01 vs. non-dyskinetic.

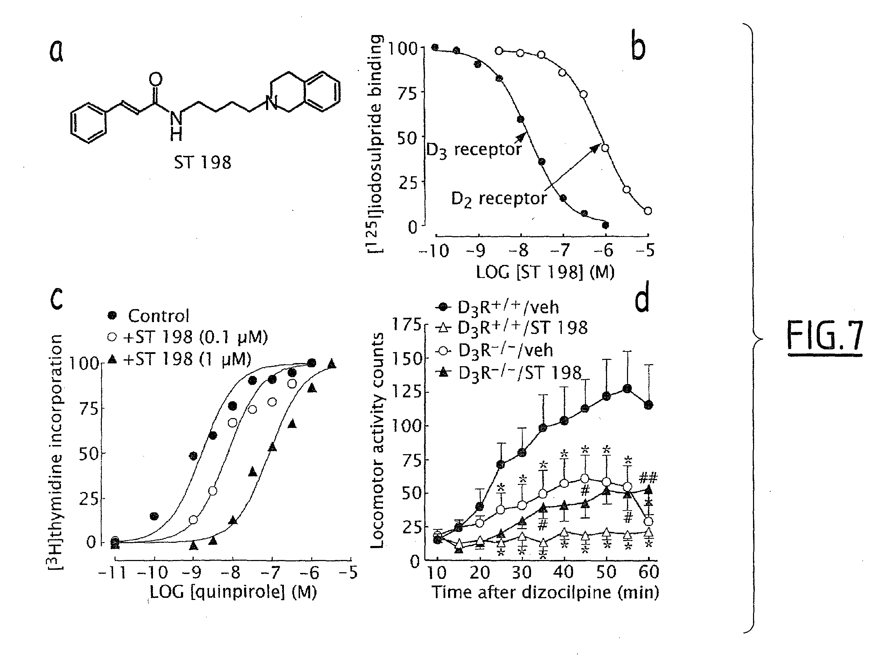

Figure 7 illustrates that ST 198 is a D₃ receptor-selective antagonist. ***a,*** Chemical structure of ST 198. ***b,*** Inhibition by ST 198 of [¹²⁵I] iodosulpride binding to recombinant D₂ and D₃ receptors. ***c,*** In NG 108-15 cells expressing the D₃ receptor, ST 198 , while having no effect by itself, reversibly antagonized quinpirole-induced mitogenesis, measured as incorporation of [³H]thymidine. Results are expressed as percentage of the maximum response induced by quinpirole (1 µM). ***d,*** Inhibition by ST 198 (30 mg/kg p.o.) of locomotor activity induced by dizocilpine (0.12 mg/kg i.p.) in wild-type (D₃R^{+/+}) and D₃ receptor gene-targeted (D₃R^{-/-}) mice. Mean ± S.E.M. of values obtained with 12 animals per group. There was a significant treatment X genotype interaction (F_{1,43} = 6.99, *P* = 0.011). * *P* < 0.01 vs. D₃R^{+/+}/vehicle (veh); § *P* < 0.05, §§ *P* < 0.01 vs. D₃R^{+/+}/ST 198.

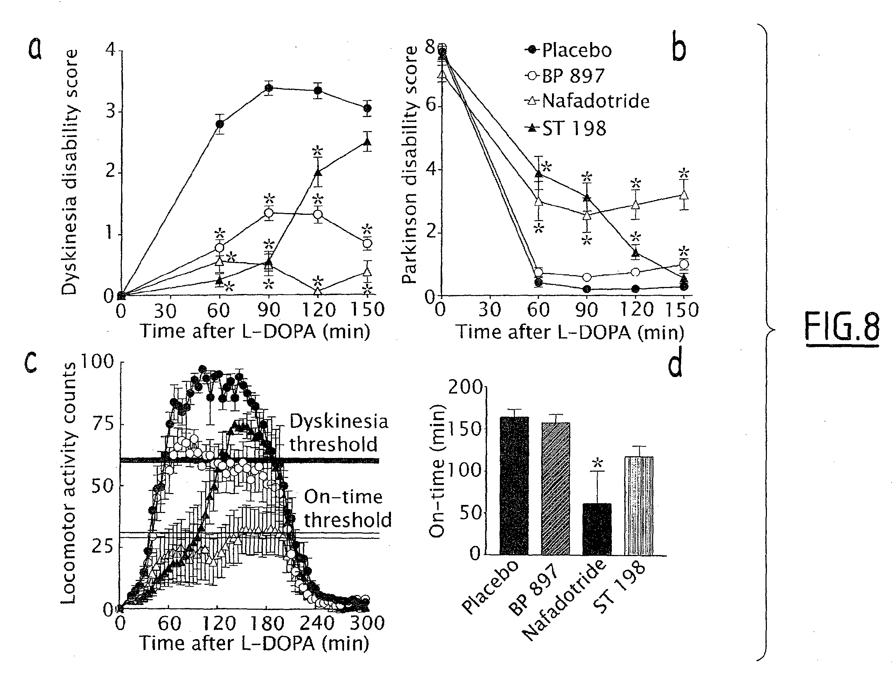

Figure 8 describes that a partial D₃ receptor agonist, but not D₃ receptor antagonists, reduced levodopa-induced dyskinesia without affecting the therapeutic effects of levodopa. ***a-c***, Levodopa-induced dyskinesia ***(a),*** PD-like symptoms ***(b)*** and locomotor activity ***(c)*** as a function of time in MPTP-intoxicated monkeys treated with either placebo, the D₃ receptor partial agonist BP 897 administered at an optimal dosage, or the D₃ receptor antagonists nafadotride or ST 198, both administered at a minimal dosage. The five MPTP-intoxicated monkeys which developed levodopa-induced dyskinesia were tested in 88 occasions (placebo, *n* = 24; BP 897, *n* = 32; nafadotride, *n* = 16; ST 198, *n* = 16). Whereas independent data points (means ± S.E.M.) of values obtained for each treatment are used for graphical representation in order to take advantage of the repetition, statistical comparison was done using the mean score obtained by each monkey (*n* = 5) for each treatment. D₃ receptor acting agents significantly affected both the dyskinesia disability score (Fr = 34.6 with 4 d.f., *P* < 0.05) and Parkinson disability score (Fr = 42.3 with 4 d.f., *P* < 0.05) over the time course (e.g. at t = 60 min, with 3 d.f., Fr = 16.8, *P* < 0.05 and Fr = 8.7, P < 0.05 for dyskinesia and Parkinson disability scores, respectively; at t = 150 min, Fr = 17.9, *P* < 0.05 and Fr = 7.1, *P* < 0.05, for dyskinesia and Parkinson disability scores, respectively). * *P* < 0.05 vs. placebo. **d**, on-time duration measured from locomotor activity counts over a 5 hr-period of time (F_{3,9} = 5.50, *P* = 0.001). * *P* < 0.05 vs. placebo.

### EXAMPLES:

### EXAMPLE 1 : Acute NMDA receptor blockade induces locomotor hyperactivity mediated by dopamine D₃ receptor

### Materials and methods

### Animals

Male Swiss mice (Iffa Credo, France), weighing 25-30 g, were housed four per cage (25 × 15 × 13 cm), on a 12 hr/12 hr light/dark cycle (lights on at 07:00 a.m.), with food and water *ad libitum.* Room temperature ranged from 20° to 22°C and humidity, thought not controlled, varied between 55% and 65%. Experiments with D₃R-deficient mice were performed on male homozygous mutant mice and their wild-type littermates. They were initially obtained from C57BL6 × 129sv hybrid heterozygous mice bearing a mutation invalidating the D₃R gene, originally obtained from S. Fuchs (Weismann Institute, Revohot, Israel, see Accili et al., 1996), that were bred, mated and genotyped as described (Perachon et al., 2000). Subsequently, these mice were backcrossed up to 8 times with C57BL6 mice. The mice used in the present study were backcrossed 7 to 8 times.

### Drugs

Dizocilpine [(+)-5-methyl-10,11-dihydro-5H-dibenzo[a,d]cyclohepten-5,10-imine maleate hereafter called MK-801] (Sigma) and nafadotride (tartrate salt, gift of C.G. Wermuth, Strasbourg, France) were dissolved in saline (0.9% NaCl). BP 897 (dihydrochloride salt, gift of C.G. Wermuth) was dissolved in water and then diluted in saline. Haloperidol (Janssen Pharmaceuticals, Beerse, Belgium) and clozapine (Novartis, Basel, Switzerland) were dissolved in a few drops of hydrochloric acid and distilled water, and the pH of the solution was adjusted to pH 5 to 6 using NaOH. The doses of BP 897, nafadotride, haloperidol and clozapine are expressed as the weight of the base. The compounds were injected i.p. in a volume of 5 ml/kg.

### Locomotor activity

All experiments were conducted between 2.00 p.m. and 6.00 p.m., and the animals were kept in the test room at least 1 h before recording their activity. The locomotor activity was measured in an actimeter that was composed of eight individual activity cages (30 × 15 × 18 cm, with sawdust on the floor) transected by infrared beams (Imetronic, Pessac, France). Counts for forward horizontal activity were incremented each time the animal moved from one-half part of the cage to the other, corresponding to disruption of two crossed parallel beams distant of 14 cm. In time-course studies, counts were summed every 5 min.

Mice were injected with either saline, BP 897 (0.1, 0.3, 1 mg/kg), nafadotride (0.1, 0.3, 1 mg/kg), haloperidol (0.03, 0.1, 0.3 mg/kg) or clozapine (0.1, 0.3, 1 mg/kg), and their horizontal locomotor activity was measured for 30 min (spontaneous locomotor activity). They were subsequently treated with saline or MK-801 (0.12 or 0.3 mg/kg) and the activity was measured for 1h (MK-801-induced locomotor activity).

Experiments using the D₃R-deficient mice were performed in the same manner with four groups of 10-12 mice of each genotype, injected with either saline, BP 897, nafadotride or clozapine (the three compounds at 1 mg/kg). This experiment was replicated once, and since the results did not differ between the two experiments, the data were pooled. In addition, another experiment was performed with two groups of 8 mice of each genotype, injected with either saline or haloperidol (0.05 mg/kg).

### Statistical analysis

Between-group differences were analyzed with the Statistica™ software using two-way analysis of variance (ANOVA) and Least Significance Difference (LSD) post-hoc test. The analysis of data obtained with the same animals evaluated on several occasions (time-course experiments), between-within univariate or multivariate ANOVA for repeated measures were used (time being the repeated measure).

### Results

### MK-801-induced locomotor activity in mice

After a 30-min period of habituation (spontaneous activity) during which the locomotor activity declined, injection of MK-801 (0.12 or 0.3 mg/kg i.p.) produced locomotor hyperactivity while the spontaneous activity continued to decline in saline-treated animals. The MK-801 treatment gradually increased activity, reaching a steady-state level after about 30 min which remained stable during at least 60 min following the injection. The response induced by MK-801 at the dose of 0.3 mg/kg was about three times that produced by this compound at the dose of 0.12 mg/kg, revealing a significant effect of dose (F_{2,36} = 14.55, P < 0.0001), time ( F_{17,612} = 10.75, P < 0.0001) and treatment × time interaction (F_{34,612} = 11.38, P < 0.0001).

At the dose of 0.12 mg/kg, which was used in most of the subsequent experiments, the response to MK-801 mainly consisted in horizontal locomotor hyperactivity. Rearing remained very low after MK-801 administration (17 ± 10 rearings during 60 min) and other stereotypies, such as licking or grooming were as low as in saline-treated animals upon visual examination. No further attempts were made to quantify these stereotypies.

### Effects of D₃ receptor antagonists and antipsychotic drugs on spontaneous and MK-801-induced locomotor activity

The effects of D₃ receptor antagonists and antipsychotic drugs were examined during two phases: the first 30-min period, starting 5 min after drug or saline injection, corresponds to habituation (spontaneous activity) and the 60-min period following injection of MK-801 at the dose of 0.12 mg/kg corresponds to locomotor activity under the influence of the compound (activity with MK-801). The D₃ receptor-selective partial agonist BP 897 (Fig. 2A) dose-dependently reduced the activity with MK-801 (black bars), without any effect during the habituation phase (white bars, i.e. spontaneous activity). There was a significant effect of dose (F_{3,42} = 2.86, P < 0.05), of phase (F_{1,42} = 16.40, P < 0.01) and dose X phase interaction (F_{3,42} = 3.54, P < 0.05). Post-hoc analysis indicated that BP 897 significantly reduced MK-801-induced hyperactivity at the doses of 0.3 mg/kg (P < 0.05) and 1 mg/kg (P < 0.01).

Nafadotride (Fig. 2B) also dose-dependently inhibited activity with MK-801, without affecting spontaneous locomotion. There was a significant effect of dose (F_{3,37} = 3.40, P < 0.05), of phase (F_{1,37} = 9.64, P < 0.01) and dose X phase interaction (F_{3,37} = 4.52, P < 0.01). Post-hoc analysis indicated that nafadotride produced a significant effect at the doses of 0.3 mg/kg (P < 0.01) and 1 mg/kg (P < 0.005).

Clozapine (Fig. 2C) produced similar effects to those observed with BP 897 and nafadotride. There was a significant effect of dose (F_{3,63} = 2.86, P = 0.04), but neither significant effect of phase (F_{1,63} = 1.75, P = 0.19) nor dose X phase interaction (F_{1,63} = 2.01, P = 0.12). Post-hoc analysis indicated that clozapine significantly attenuated MK-801-induced hyperactivity at the doses of 0.3 mg/kg (P < 0.01) and 1 mg/kg (P < 0.005), while it had no effect on spontaneous activity at any of the doses tested.

In contrast, haloperidol (Fig. 2D) strongly and dose-dependently blocked the stimulatory effect of MK-801, but it was accompanied by a reduction of the spontaneous activity. There was a significant effect of dose (F_{3,47} = 8.94, P < 0.0001), but neither significant effect of phase (F_{1,47} = 0.61, P = 0.44) nor significant dose X phase interaction (F_{3,47} = 2.31, P = 0.09). Post-hoc analysis revealed that haloperidol significantly reduced spontaneous activity at the dose of 0.3 mg/kg (P < 0.05) and MK-801-induced hyperactivity at the doses of 0.03 mg/kg (P < 0.05), 0.1 mg/kg (P < 0.01) and 0.3 mg/kg (P < 0.005).

The ability of BP 897 or nafadotride (both at 1 mg/kg) to inhibit locomotor hyperactivity induced by MK-801 at the dose of 0.3 mg/kg was also tested. BP 897 and nafadotride decreased the activity in the presence of MK-801 by 10-15%, but these effects were statistically non significant (F_{1,27} = 2.31, P = 0.14 and F_{1,27} = 2.93, P = 0.10, for BP 897 and nafadotride, respectively).

### MK-801-induced activity and its inhibition by D₃ receptor antagonists and antipsychotic drugs in D₃ receptor-deficient mice

Fig. 3A represents the temporal effect of MK-801 (0.12 mg/kg) on wild-type (D₃R^{+/+}) and D₃ receptor-deficient (D₃R^{-/-}) mice, compared to their respective saline-treated control animals. In D₃R^{+/+} mice, MK-801 produced a hyperactivity that was comparable to that previously observed in Swiss mice. In D₃R^{-/-} mice, MK-801 also increased locomotor activity (significantly higher than that in saline-treated animals at time points > 45 min), but the stimulatory effects of MK-801 were strongly attenuated. The locomotor activity after saline treatment did not differ between D₃R^{+/+} and D₃R^{-/-} mice. There was a significant effect of treatment (F_{1,36} = 21.79, P < 0.01), of genotype (F_{36,1} = 5.81, P < 0.05) and treatment X genotype interaction (F_{36,1} = 4.28, P < 0.05).

As in Swiss mice, BP 897, nafadotride or clozapine, all at the dose of 1 mg/kg, inhibited MK-801-induced activity in D₃R^{+/+} mice (Fig. 3B). Neither of the drugs affected spontaneous activity during the habituation phase. The three compounds reduced the activity with MK-801 to a level equivalent to the activity in saline-treated mice in Fig. 3A, suggesting a complete blockade of the effects of MK-801. Furthermore, BP 897 and nafadotride were no longer able to inhibit the locomotor response to MK-801 in D₃R^{-/-} mice, whereas clozapine kept an inhibitory effect on these mice (Fig. 3B). Two-way analyses of variance, performed with the whole data set indicate no overall effect of genotype (F_{1,172} = 0.002, P = 0.964), a significant effect of treatment (F_{1,172} = 17.17, P < 0.00001) and genotype X treatment interaction (F_{1,172} = 8.34, P < 0.0001). Since the effect of clozapine seemed to differ from those of other compounds, we also performed two-way analyses of variance with each group of data (the same saline-treated control group was used). These analyses revealed a significant effect of BP 897 (F_{1,89} = 16.13, P < 0.01), nafadotride (F_{1,89} = 16.02, P < 0.01) and clozapine (F_{1,99} = 28.48, P < 0.01). A significant effect of genotype is only observed in the case of data with clozapine (F_{1,99} = 5.68, P < 0.05). Nevertheless, there was a significant genotype X treatment interaction for the three compounds (F_{1,89} = 16.13, P < 0.01 for BP 897; F_{1,89} = 16.02, P <0.01 for nafadotride; and F_{1,99} = 5.84, P < 0.05 for clozapine).

Haloperidol (0.05 mg/kg) was tested in a similar, but distinct experiment (Fig. 3C). MK-801-induced locomotor activity was strongly inhibited by haloperidol in D₃R^{+/+} mice and attenuated in D₃R^{-/-} mice. There was no effect of genotype (F_{1,28} = 3.52, P = 0.071), but a significant effect of treatment (F_{1,28} = 1.06, P < 0.01) and a significant treatment X genotype interaction (F_{1,28} = 5.11, P < 0.05).

A higher dose of MK-801 (0.3 mg/kg) was also tested on D₃R^{+/+} and D₃R^{-/-} mice. There were no significant differences in the stimulatory effects of MK-801 at this dose between D₃R^{+/+} and D₃R^{-/-} mice (no significant effect of genotype, F_{1,37} = 0.39, P = 0.54). Moreover, the minimal active dose of BP 897 to reduce the hyperactivity induced by 0.3 mg/kg of MK-801, was 3 mg/kg. At this dose, BP 897 had the same effects in D₃R^{+/+} and D₃R^{-/-} mice (significant effect of BP 897, F_{1,37} = 20.27, P < 0.0001; no significant BP 897 X genotype interaction, F_{1,37} = 0.53, P = 0.47).

These results establish, using D₃ receptor-selective ligands and D₃ receptor-deficient mice, that the D₃ receptor mediates locomotor hyperactivity produced by an acute treatment with a low dose of MK-801. This response constitutes the first simple behavioral test for the *in vivo* assessment of D₃ receptor-selective compounds activity. Moreover, these data indicate that D₃ receptor blockers produce the same effects as antipsychotic drugs, which may suggest a common mechanism of action.

Hyperactivity induced by a low dose of MK-801 (0.12 mg/kg) is inhibited by BP 897 and nafadotride, D₃ receptor-preferring ligands, at the doses of 1 mg/kg or lower, which produce a selective D₃ receptor occupancy (Levant and Vansell, 1997; Pilla et al., 1999). Whereas nafadotride is a neutral D₃ receptor antagonist (Sautel et al., 1995), BP 897 is a partial agonist *in vitro* and acts *in vivo* as either an agonist, when dopamine is depleted, or as an antagonist when the receptor is subject to a sustained stimulation by dopamine (Pilla et al., 1999). The latter situation is likely to occur in the present experiments, since MK-801-induced hyperactivity is mediated by an enhancement of dopamine function produced by systemic administration of NMDA receptor non-competitive antagonists. The similar effects of BP 897 and nafadotride on MK-801-induced hyperactivity therefore suggest that both compounds act by blocking overstimulated postsynaptic D₃ receptors. Nevertheless, it cannot be excluded that BP 897, as a partial agonist, concomitantly stimulates D₃ autoreceptors, localized on all dopamine neurons of the mesencephalon (Diaz et al., 2000) and whose function on dopamine release has recently been demonstrated (Zapata et al., 2001). Moreover, dopamine autoreceptors are more sensitive to partial agonists than postsynaptic receptors (Meller et al., 1986). Conceivably, BP 897 may reduce MK-801-induced dopamine hyperfunction by both inhibiting dopamine neuron activity via the stimulation of autoreceptors, and antagonizing dopamine postsynaptic receptors.

Furthermore, D₃ receptor-deficient mice displayed two to three times less MK-801-induced hyperactivity as their wild-type littermates and inhibition produced by both BP 897 and nafadotride was completely abolished. These results demonstrate the involvement of D₃ receptors in the response to MK-801 and the D₃ receptor selectivity of BP 897. The results obtained with nafadotride are surprising since the effect of this compound (at 0.03-3 mg/kg) was previously shown to persist in D₃R^{-/-} mice, and therefore, not to be D₃ receptor-dependent (Xu et al., 1999). These results favor the use of a NMDA receptor response, such as the MK-801 response, which appears more sensitive for the prediction of the *in vivo* potency of D₃ receptor-selective drugs.

When using the lowest dose of MK-801 (0.12 mg/kg), BP 897 and nafadotride reduced locomotor activity in MK-801-treated mice to a level (30-40 %) corresponding to that displayed by saline-treated mice (shown for instance in Fig. 1 and 3). Since neither of the two compounds inhibit spontaneous activity (Pilla et al., 1999; Sautel et al., 1995 and present study), this suggests that hyperactivity induced by MK-801 at the lowest dose is largely D₃ receptor-dependent.

In contrast, hyperactivity produced by MK-801 at 0.3 mg/kg, which is three times higher than that produced by this compound at the lowest dose, is similar in D₃R^{+/+} and D₃R^{-/-} mice and only partially and non significantly inhibited by either BP 897 or nafadotride.

Non-dopaminergic drugs acting at various receptor types are able to reduce locomotor activity induced by MK-801, generally used at doses higher than those in the present study (Andiné et al., 1999). These include ritanserin, a 5HT₂ receptor antagonist (O'Neill et al., 1999) and prazosin, an α1-adrenergic antagonist (Mathé et al., 1996). This suggests that NMDA receptor blockade can produce locomotor hyperactivity by interfering with systems controlling dopamine systems, such as the serotoninergic and noradrenergic systems (Grenhoff et al., 1993; Prisco and Esposito, 1995), or recruit other neurotransmitter systems. The latter hypothesis likely accounts for the MK-801-induced hyperactivity observed in D₃R^{-/-} mice, which is insensitive to BP 897 and nafadotride, whereas it is almost completely sensitive in wild-type mice. Thus, the MK-801-induced hyperactivity in D₃R^{-/-} mice is D₃ receptor-independent and might result from a mechanism compensating for the constitutive lack of D₃ receptors, and presumably, of stimulatory receptors on locomotion. This hypothesis is consistent with previous observations of a locomotor stimulatory role of MK-801 in monoamine-depleted animals (Carlsson and Carlsson, 1989). This latter effect, however, was obtained with high doses of MK-801 (> 1 mg/kg), was accompanied with ataxia and is likely to contribute for a very limited part in the effects of low doses of MK-801 used in the present study.

Hence our results indicate that activation of the D₃ receptor is a major mechanism underlying locomotor effects of NMDA receptor blockers at low doses. These effects mainly consist in a mild enhancement of horizontal locomotion, with no other stereotypies, which is consistent with previous observations (Ninan and Kulkarni, 1999). A locomotor stimulatory role of D₃ receptors has already been suggested for mediating dopamine agonist-induced rotations in hemiparkinsonian rats (Bordet et al., 1997) and cocaine-conditioned hyperactivity (Le Foll et al., 2002). The D₃ receptor, however, seems to take only a minor part in the maintenance of spontaneous locomotor activity, which is not affected by D₃ receptor-selective partial agonist and antagonists (present study; Reavill et al., 2000; Millan et al., 2000). This suggests that the D₃ receptors mediating locomotion are largely unoccupied by dopamine under basal conditions, which contradicts previous conclusions that are based, however, on results obtained with an indirect method for measuring brain D₃ receptor occupancy (Schotte et al., 1992).

The measurement of D₃ receptor-selective blockade *in vivo* has been previously based on complex and laborious tests, such as inhibition of rotations in levodopa-primed hemiparkinsonian rats (Bordet et al., 1997; Pilla et al., 1999), study of induction of *c-fos* expression in very small brain structures by *in situ* hybridization analysis (Pilla et al., 1999) or correction of isolation-induced disruption of prepulse inhibition (Reavill et al., 2000). The results provided here show that hyperactivity produced by MK-801 at a low dose can be used as a very simple response mediated by the D₃ receptor to assess the *in vivo* activity of D₃ receptor-selective compounds, which have no effects on spontaneous activity (Millan et al., 2000; Reavill et al., 2000).

The effects of haloperidol and clozapine, which are typical and atypical antipsychotic drugs, respectively, were further examined to gain information in the potential involvement of D₃ receptor blockade in the therapeutic effect of antipsychotic drugs. In this context, it was of interest to examine Haloperidol is a rather selective D₂/D₃/D₄ receptor blocker, whereas clozapine has multiple targets, in addition to its dopaminergic targets (Meltzer, 1994). Both clozapine and haloperidol inhibited the response to MK-801, in agreement with previous studies (Jentsch and Roth, 1999; Murray and Horita, 1979; Ögren and Goldstein, 1994). Haloperidol produced the strongest inhibition when compared to other compounds, but also inhibited spontaneous activity during habituation. The latter response is probably due to D₂ receptor blockade by haloperidol, since D₂/D₃ receptor antagonists, but neither D₃ receptor-selective antagonists (Millan et al., 2000; Reavill et al., 2000) nor clozapine (Schaefer and Michael, 1984) produce hypolocomotion. The effect of clozapine was strikingly similar to those produced by BP 897 and nafadotride in this respect.

In order to investigate whether clozapine and haloperidol directly interferes with D₃ receptor signalling, the effects of the two compounds was assessed on MK-801-induced hyperactivity in D₃R^{-/-} mice. Like BP 897 and nafadotride, clozapine or haloperidol completely inhibited MK-801-induced hyperactivity in D₃R^{+/+} mice. However, in contrast with the two former compounds, clozapine significantly inhibited hyperactivity in D₃R^{-/-} mice. The inhibition of MK-801-induced by haloperidol in D₃R^{-/-} mice was non significant and there was a statistically significant treatment X genotype interaction for both clozapine and haloperidol, which may suggest that the effects of these two compounds were lesser in D₃R^{-/-} than in D₃R^{+/+} mice. However, since the MK-801-induced hyperactivity in D₃R^{-/-} mice was lower than in D₃R^{+/+} mice, this statistical interaction may in fact, reflect a "floor" effect: the activity measured after treatment with clozapine or haloperidol might have been lowered to the normal activity. The effect of clozapine in D₃R^{-/-} mice is likely to occur by a D₃ receptor-independent mechanism, upregulated by D₃ receptor knockout (see above), via interaction of the various receptors targeted by this compound.

Thus, the experiments conducted with D₃ receptor-deficient mice allow to conclude that the effects of BP 897 and nafadotride are most likely the result of a selective interaction with the D₃ receptor. The same conclusion cannot be drawn with clozapine and haloperidol, because of the lateral, non-D₃ receptor binding properties of these compounds. Clozapine and haloperidol might inhibit MK-801-induced hyperactivity by either blocking the D₃ receptor, or interfering with the chain of neural events initiated by NMDA receptor blockade and leading to locomotor activity. In support of the former hypothesis, both clozapine and haloperidol display high affinity for the D₃ receptor, yet slightly lower than that for the D₂ receptor (Sokoloff et al., 1990).

Taken together, these results show that hyperactivity produced by the NMDA receptor antagonist MK-801 at a low dose, likely to be equivalent to the dose of PCP or ketamine producing psychodysleptic effects in humans, is highly dependent on D₃ receptor stimulation. Blockade of the D₃ receptor produces an effect very similar to that produced by antipsychotics on MK-801-induced hyperactivity, which suggests that D₃ receptor blockers may have antipsychotic properties.

### EXAMPLE 2: Chronic NMDA receptor blockage induces locomotor activity mediated by dopamine D₃ receptor

### Material and methods

### Animals

Male Swiss mice (IFFA CREDO, France), weighing 26-28 g, were housed six per cage (25 × 15 ×13 cm), on a 12 h/12 h light/dark cycle (lights on at 07:00 a.m.), with food and water freely available. Room temperature ranged from 20° to 22°C and humidity varied between 55% and 65%.

### Treatment with MK-801

Dizocilpine, as (+)-MK-801 maleate (Sigma), dissolved in 0.9% NaCl (saline), and its respective vehicle solution, was continuously infused via osmotic minipumps (Model 1007D, Alzet Corp.). Minipumps were filled 6 h prior to implantation and placed in a sterile saline water bath at 37°C. Taking into consideration the average weight of the mice at the time of testing, and the rate of the minipump delivery (specified by the manufacturer), the MK-801 concentration was adjusted to deliver 0.01 or 0.02 mg/kg/hr (0.48 mg/kg/day). Mice were anaesthetized with chloral hydrate (400 mg/kg i.p.), and an area on the back of the mice was shaved and cleaned with 70% ethanol. A small incision was made, a pocket beneath the skin was formed and the minipump was placed inside the pocket in each mouse. The incision was sutured, and cleaned with 70% ethanol. The mice were returned in their home cage and in about 2 h they had recovered and were active.

### Locomotor activity

Once the minipumps implanted (Day 0), the locomotor activity was measured for 1h, at days 2, 4 and 7 in an actimeter (Imetronic, Pessac, France). Haloperidol (Janssen Pharmaceuticals, Beerse, Belgium), clozapine (Novartis, Basel, Switzerland) or BP 897 (Bioprojet, France) was given either acutely at day 10 or 11. In some experiments, haloperidol or BP 897 was given twice a day for 4.5 days beginning at day 6.

### Resident-intruder assay

Male mice were housed individually or in groups of five for at least 1 week prior to minipump implantation (Day 0), delivering MK-801 (0.02 mg/kg/h) or saline (Sham), and were tested at day 7. As described in Dixon et al. (1994), a group-housed male (intruder) was placed in the home cage of the individually housed male (resident), and their behaviors were video recorded for 5 min. Thirty min prior testing, saline, BP 897 (1 mg/kg i.p.) or clozapine (1 mg/kg i.p.) were administered to resident and intruder mice. Experimental groups included groups of 10 residents receiving sham + saline, MK-801 + saline, MK-801 + BP 897 residents, or MK-801 + clozapine, and their respective groups of intruders. The videotaped behaviors of the resident or intruder male were individually scored, by an experimenter unaware of the treatment group, for time spent in social investigation (approaching, sniffing, grooming other mouse, and sexual behavior), and in escape behavior (actively avoiding the unfamiliar congener). The scores for each behavior were subsequently averaged for each group.

### Statistical analysis

Between-group differences were analyzed with the Statistica™ software using two-way analysis of variance (ANOVA) and Least Significance Difference (LSD) post-hoc test. The analysis of data obtained with the same animals evaluated on several occasions (time-course experiments), between-within univariate or multivariate ANOVA for repeated measures were used (time being the repeated measure). Data from the resident-intruder assay were analyzed by analysis of variance (ANOVA), followed by a Tukey-Kramer Multiple Comparison post-hoc test.

### Results

A chronic infusion through osmotic minipumps for 7 days with MK-801 produced in mice a locomotor hyperactivity, which first appeared after 7 days of treatment and lasted at least 4 days after the date of cessation of the drug delivery, as assumed from the osmotic minipump manufacturer's datasheet (Fig. 4). The effect of MK-801 was observed at a dose of 0.01 mg/kg/h and was more pronounced at a dose of 0.02 mg/kg/h. The spontaneous hyperactivity progressively developing upon chronic infusion with MK-801 may mimic agitation in patients with schizophrenia.

Clozapine (1 mg/kg) or haloperidol (0.3 mg/kg), atypical and typical antipsychotic drugs, respectively, inhibited MK-801-induced hyperactivity, when administered acutely on the 10^{th} day following minipumps implantation (Fig. 4b). BP 897 (1 mg/kg), a highly D₃ receptor-selective partial agonist (Pilla et al., 1999), produced an effect similar to that produced by clozapine or haloperidol (Fig. 4b), two D₂/D₃ receptor blockers. The effects of antipsychotic drugs on MK-801-induced hyperactivity may mimic their immediate therapeutic effects on positive symptoms, such as agitation and hallucinations.

That MK-801-induced hyperactivity required stimulation of the D₃ receptor was confirmed by using D₃ receptor gene-targeted (D₃^{-/-}) mice, in which MK-801-induced hyperactivity was attenuated by ~50% when compared to wild-type (D₃^{+/+}) mice (Fig. 4c). The treatment by MK-801 also induced in mice a state of hypersensitivity to methamphetamine, a psychostimulant, the locomotor response of to which was enhanced in MK-801-treated mice (Fig. 4d). The hypersensitivity to methamphetamine was completely abolished in D₃^{-/-} mice (Fig. 4d), indicating that it requires D₃ receptor stimulation. Hypersensitivity to psychostimulants is presented by schizophrenic patients, whose psychotic symptoms are exacerbated by psychostimulants at doses inactive in normal individuals (Carlsson, 1988) and in whom psychostimulant-induced dopamine release is abnormally elevated (Laruelle, 2000).

The social interactions between MK-801-treated animals were investigated using a resident-intruder assay, which can quantify "approach-oriented" behavior and social withdrawal (Corbett et al., 1993; Dixon et al., 1994). The treatment with MK-801 elicited an enhancement of escape behavior, which was completely normalized by clozapine or BP 897 (Fig. 4e). Social investigations were reduced by the treatment with MK-801, and restored by clozapine or BP 897 (Fig. 4f). These deficits in social interactions mimic social withdrawal in patients with schizophrenia.

These results show that a chronic infusion with a low dose of the NMDA antagonist MK-801 produced behavioral abnormalities mimicking symptoms of schizophrenia, which were circumvented by D₃ receptor knockout or by D₃ receptor blockade. Since the behavioral abnormalities were prominent and persisted after discontinuation of the MK-801 treatment, it was hypothesized that MK-801 produced enduring alterations of brain functions. It was determined whether a subchronic treatment with an antipsychotic or a D₃ receptor blocker was able to correct these alterations. For this, a treatment with either haloperidol or BP 897 (twice a day) was initiated at the emergence of the abnormalities (6 days following the beginning of MK-801 delivery) and continued for 4.5 days. Spontaneous and methamphetamine-induced locomotor activities were recorded at least 6 hours after the last administration of haloperidol or BP 897 at a time when the acute effects of these drugs have disappeared. The subchronic treatment with haloperidol or BP 897 reversed both spontaneous (Fig. 5a) and methamphetamine-induced (Fig. 5b) hyperactivity. These results show that the subchronic treatments reversed the alterations induced by MK-801.

Taken together, these results indicate that the D₃ receptor participates in the generation of behaviors abnormalities elicited by chronic NMDA receptor blockade. These abnormalities mimic symptoms of schizophrenia, notably negative symptoms, and are circumvented by acute or chronic administration of a D₃ receptor-selective blocker, or by antipsychotic drugs that are D₂/D₃ receptor blockers. Therefore, the model presently described can serve as a simple test for predicting the antipsychotic potential of D₃ receptor ligands, administered either acutely or chronically.

### EXAMPLE 3: Identification of a novel selective inhibitor of D₃ receptor

In the course of a pharmacochemistry program aiming at the discovery of novel D₃ receptor antagonists, ST 198 ((E)-*N*-(4-[1,2,3,4-tetrahydroisoquinolin-2-yl]-butyl)-3-phenylacrylamide, see Fig. 7a) was designed and synthesized. A short note on ST 198 has been published (Weber et al., 2001). Initial screening was performed using human recombinant D₂ and D₃ receptors as described (Pilla et al., 1999). Further binding screening was performed by CEREP (Celle l'Evescaut, France).

ST 198 had inhibition constants (Kᵢ) of 12 ± 0.5 nM and 780 ± 30 nM (*n* = 4) for inhibiting binding to D₃ and D₂ receptors, respectively (Fig. 7b), and a lower affinity for human D₁ (Kᵢ ~ 25 µM), D_{4.4} (Kᵢ ~ 5 µM), and D₅ receptors (Kᵢ ~ 12 µM), as well as for a variety of non-dopaminergic receptors (Kᵢ > 1 µM), including adenosine A₁ (Kᵢ ~ 5 µM) and A_{2A} (Kᵢ > 20 µM), noradrenergic (β₁₋₃, except β₁ and β₂ receptors, Kᵢ = 100 nM), serotonergic (except 5HT_{1A}, Kᵢ = 240 nM), muscarinic M₁₋₂, µ opioid and histaminergic H₁₋₃ receptors. The intrinsic activity of ST 198 was measured using the mitogenic response in the NG 108-15 cell line engineered to express recombinant human D₃ receptors (Pilon et al., 1994) (Fig. *7c*): ST 198 (0.1 and 1 µM) was devoid of any agonistic activity and competitively antagonized the response produced by the dopamine agonist quinpirole (pA₂ = 7.5, determined by the Schild plot).

To assess simply the *in vivo* biological activity of the novel compound at the D₃ receptor, the above described test, in which hyperlocomotion induced by low doses of dizocilpine is measured, was used. *In vivo* activity was measured as the ability of the compound administered intraperitonally to inhibit locomotor activity in Swiss mice, measured during 60 min after administration of dizocilpine (0.12 mg/kg i.p., injected 30 min after ST 198).

ST 198 inhibited dizocilpine-induced hyperlocomotion (ID₅₀ = 25 mg/kg) and this effect was abolished in D₃ receptor-deficient mice (Fig. 7d).

These results demonstrate that the effect of ST 198, at the doses used, indeed resulted from D₃ receptor blockade.

### EXAMPLE 4 : levodopa-induced dyskinesia is attenuated by normalizing dopamine D₃ receptor function

### Materials and Methods

### Animals

Experiments were conducted on sixteen female Cynomolgus monkeys (Macaca fascicularis, SAH, Beijing, PR of China) according to procedures and methods previously published (Suzuki et al., 1998). Four monkeys were kept normal (control group). The remaining twelve were intoxicated with MPTP hydrochloride. Once a bilateral parkinsonian syndrome had stabilised (i.e. unchanged disability score over several weeks), three monkeys were kept without any dopaminergic supplementation (MPTP group) and the nine remaining parkinsonian monkeys were treated chronically with twice daily administration of levodopa (Modopar®, L-DOPA/carbidopa, ratio 4:1) for 6-8 months at a tailored dose designed to produce a full reversal of parkinsonian condition (47 to 60 mg/kg of levodopa). On these nine monkeys, five developed severe dyskinesia of a predominantly choreic nature, which were then reproducible to subsequent doses of levodopa/carbidopa (MPTP-intoxicated, dyskinetic monkey group), while four did not (MPTP-intoxicated, non-dyskinetic monkey group). After the MPTP-intoxicated dyskinetic monkeys have been enrolled in the pharmacological trial, four of them have been treated with levodopa alone for 3 weeks before euthanasia.

PD-like rats were obtained after a unilateral lesion of dopamine neurons by infusion of 6-hydroxydopamine and treatment with the combination of levodopa and benserazide (50 + 12.5 mg/kg i.p.) twice a day for 10 days as described (Bordet et al., 1997).

### Behavioral assessment

Parkinsonian condition (and reversal) was assessed on a parkinsonian monkey rating scale using videotape recordings of monkeys in a dedicated cage and clinical neurological evaluation as previously described (Suzuki et al., 1998 ; Imbert et al., 2000). A score of 0 corresponds to a normal animal and a score above 6 to a parkinsonian animal. The severity of dyskinesia was rated using the Dyskinesia Disability Scale that focuses on the issue of disability caused by dyskinesias rather than the amount of dyskinesia (Pearce et al., 1995). Thus dyskinesia was assessed as being 0, absent; 1, mild, fleeting, and rare dyskinetic postures and movements; 2, moderate, more prominent abnormal movements, but not interfering significantly with normal behaviour; 3, marked, frequent and, at times, continuous dyskinesia intruding on the normal repertoire of activity; or, 4, severe, virtually continuous dyskinetic activity, disabling to the animal and replacing normal behaviour. Locomotor activity was monitored in the dedicated cage fitted with infrared activity monitors, providing a mobility count every 5 min. Activity data were further analysed to assess "on-time" duration, defined as the total time that activity counts were above 30% of the maximum activity score (Grondin et al., 2000) over the five-hour duration of the experiment. In the placebo situation, the observation of a correlation between clinically-assessed LID appearance and a certain level of locomotor activity counts allows to define a dyskinesia threshold, based on the measurement of locomotor activity (see Fig. 7c). This threshold also corresponded to the normal activity of naive monkeys.

Rotations in PD-like rats were measured during 5 min, 40 min after administration of levodopa and BP 897, both intraperitonally (Bordet et al., 1997).

### Biochemical procedures

Dopamine transporter binding was measured using [¹²⁵I]-(E)-*N*-(3-iodoprop-2-enyl)-2β-carboxymethyl-3β-(4'-methylphenyl)-nortropane as previously described (Bezard et al., 2001). Densitomeric analysis of autoradiographs, performed using an image analysis system as described (Levesque et al., 1995 ; Bezard et al., 2001), assessed binding at three rostrocaudal levels in accordance with the functional organization of the striatum: a rostral level including the Cd, Pu and nucleus accumbens (A21.0); a mid-level including the caudate, putamen and GPe (A17.2); and a caudal level including the body of the caudate, the putamen and both parts of the GPi (i.e. pars externals and pars internals) (A14.6). Where appropriate, both caudate and putamen were divided into dorsolateral, dorsomedial, ventrolateral and ventromedial quadrants for analysis. Four sections per animal, per striatal level were analyzed by an examiner blind with regard to the experimental condition. D₃ receptor binding was measured using [¹²⁵I]-R-(+)*trans-*7-hydroxy-2-[*N*-propyl-*N*-(3'-iodoprop-2-enyl)amino]tetralin in slices at the caudal level shown in Fig. 6. Plasma concentrations of compounds were measured using a radioreceptor assay with human recombinant D₃ receptors (Pilla et al., 1999).

### Pharmaceutical trials

Animals received levodopa/carbidopa concomitantly administered with either placebo (water), BP 897 (dissolved in water, administered orally), nafadotride (dissolved in NaCl 0.9%, administered intramuscularly) or ST 198 (dissolved in water, pH 6.3, administered orally). Clinical assessments were made at 0 min (i.e. pre-administration - OFF score), 60, 90, 120 and 150 min post-administration of the compounds. Equality of distributions of pre-administration disability scores (at t = 0 min) in the four treatments was tested for each monkey and then for all animals (Kolmogorov-Smirnov equality of distribution test). All data were normally distributed (Skewness-Kurtosis test for normality). The lack of significant differences supports the fact that the parkinsonian syndrome was comparable between animals and remained stable throughout the experiments.

### Statistical analysis

One-way or three-way ANOVA was used to estimate overall significance for multiple comparisons of binding data, an ANOVA for repeated measures was used for comparison of on-time durations, both followed by post-hoc *t* tests corrected for multiple comparisons by the method of Bonferroni. For multiple comparisons of behavioural assessments, Friedman non-parametric repeated measures test was used to estimate overall significance, followed by two-by-two comparisons using the Wilcoxon matched pairs signed-rank test. All data were normally distributed (Skewness-Kurtosis test), and significance levels of *t* test comparisons were adjusted for inequality of variances when appropriate. These analyses were completed using the STATA program (Intercooled Stata 6.0, Stata Corporation, College Station, TX). A probability level of 5% (*P* < 0.05) was considered significant.

### Results

In a rat model of PD (PD-like rats), D₃ receptor expression is ablated (Levesque et al., 1995), but repeated administration of levodopa increases motor responses to this drug, by markedly and gradually inducing D₃ receptor gene expression in the caudate-putamen, a brain region involved in motor control (Bordet et al., 1997). Induction of the D₃ receptor gene is likely to occur in the caudate-putamen of PD patients treated with levodopa and may be responsible for the development of levodopa-induced dyskinesia (LID) in PD patients (Cotzias et al., 1969). Hence, pharmacological manipulations of the D₃ receptor could be beneficial for treating dyskinesia. This could not be assessed in PD-like rats, which do not develop typical LID.

Administration of MPTP to monkeys destroys dopamine terminals and cell bodies and produces a variety of PD-like symptoms, including akinesia and rigidity (Burns et al., 1983). Nine MPTP-intoxicated monkeys displaying a stable parkinsonism received levodopa for several months at a tailored dose, designed to produce a full reversal of parkinsonian condition. Five out of nine levodopa-treated MPTP-intoxicated monkeys developed severe LID (score of 3.4 ± 0.1 on a maximum of 4), which were absent or minimal (score < 0.4) in the four remaining animals. The monkeys with LID exhibited various combinations of choreic-athetoid (i.e. characterized by constant writhing and jerking motions), dystonic and even ballistic movements (*i.e*. large-amplitude flinging, flailing movements), although less frequently for those latter. Both the repertoire and severity of dyskinesia were not distinguishable from LID occurring in PD patients. At the peak-dose of levodopa (80-150 min after administration), dystonic rolling and writhing on cage floor were common. The dyskinesia disability scale presently used is similar to the scale introduced into the clinics and focuses on the issue of the disability caused by dyskinesia rather than the amount of dyskinesia (Pearce et al., 1995). Thus, the score reflected the continuous dyskinetic activity that replaced the normal behavior, while the parkinsonian syndrome was fully reversed (the score was 0.2 ± 0.1, whereas parkinsonism corresponds to a score above 6).

D₃ receptor expression was compared in four out of five dyskinetic monkeys after they were used in the pharmacological trials described below, with that in normal (*n* = 4), MPTP-intoxicated monkeys never being in contact with a dopaminergic agent (*n* = 3), and the MPTP-treated monkeys that have received levodopa but have not developed LID (*n* = 4). Since the extent of nigrostriatal degeneration is an obvious variable that may play a role in the susceptibility to develop LID, we first assessed whether all MPTP-intoxicated monkeys had similar loss in striatal dopamine nerve endings by measuring dopamine transporter (DAT) binding in the caudate nucleus (Cd) and putamen (Pu) at three rostrocaudal levels, with each brain region divided in four quadrants, in accordance with the functional organization of the striatum (See Methods). Whichever the level or quadrant was considered in both Cd or Pu, DAT binding was significantly and similarly decreased in MPTP-monkeys, non-dyskinetic levodopa-treated MPTP-monkeys and dyskinetic levodopa-treated MPTP-monkeys. For example, in the dorsolateral quadrant at the rostral level, DAT binding was decreased by 96.3 ± 0.3 %, 96.7 ± 0.4 % and 95.7 ± 0.8 %, respectively, with respect to controls in Cd and by 97.1 ± 0.3 %, 97.0 ± 0.4 % and 95.0 ± 1.3 %, respectively, in Pu. Very similar results were obtained at other anteriority levels, so that there were no significant differences in the decreases in DAT binding in all MPTP-treated groups (F_{53,215} = 1.08, P = 0.35 by three-way analysis of variance, with level, quadrant and group as variables; there was no group effect when only this variable was considered: F_{2,215} = 1.61, P = 0.21). Thus, according to our present experimental design, the occurrence of dyskinesia was not related to lesion severity.

D₃ receptor binding was measured in the brain basal ganglia involved in motor control, e.g. Cd, Pu, external segment of globus pallidus (GPe) and internal segment of globus pallidus (GPi). MPTP alone produced a severe loss of D₃ receptor binding in Cd (- 68%, *P* < 0.05), a brain structure involved in associative locomotion, which was compensated by treatment with levodopa in MPTP-intoxicated animals without LID (Fig. 6). These results fully agree with previous observations in PD-like rats and MPTP-intoxicated monkeys. However, in MPTP-intoxicated monkeys with LID, D₃ receptor binding was higher than in non-dyskinetic monkeys in Pu (+ 154%, *P* < 0.05) and GPi (+ 425%, *P* < 0.01) and even higher than in normal monkeys (+ 79%, *P* <0.05 and + 116%, *P* < 0.05 in Pu and GPi, respectively). Moreover, D₃ receptor binding levels in Pu correlated with occurrence and severity of LID (r² = 0.50, *P* < 0.05, *n* = 8). These results show that PD-like symptoms and LID were accompanied by down- and upregulation of the D₃ receptor, respectively, while such a correlation does not exist for either D₁ or D₂ receptors in comparable experimental conditions (Bezard et al., 2001). In patients with PD, both decreased and unchanged D₃ receptor levels were reported. The results described above can now explain this discrepancy, because D₃ receptor expression may depend on the patients' clinical status, notably with respect to dyskinesia, which was not specified in either of the two studies.

The changes in D₃ receptor expression are likely to reflect fluctuations of D₃ receptor function, as it is the case in PD-like rats in which they are triggered primarily by down- and up-regulation of the brain-derived neurotrophic factor (BDNF) in dopamine and corticostriatal neurons, respectively, and are responsible for alterations of motor responses. Together with the data obtained with PD-like rats, the present results suggested that limiting fluctuations of D₃ receptor function could attenuate LID, without ablating motor recovery produced by levodopa.

To test this idea, the D₃ receptor-selective ligand BP 897 was used. BP 897 acts as a partial agonist *in vitro* at recombinant human D₃ receptor and *in vivo* in rodents, *i.e*. is able to act as either an agonist or an antagonist, depending on the response considered (Pilla et al., 1999). For instance, in PD-like rats repeatedly treated with levodopa, BP 897, acting as an agonist in combination with a D₁ receptor agonist, produces rotations, an index of the motor response in these rats. To further assess the partial antagonistic activity of BP 897, we administered this compound to PD-like rats in increasing dosage in combination with levodopa at a high dose (50 mg/kg). The numbers of rotations were 108 ± 7 (mean ± S.E.M., *n* = 18) in animals receiving saline, and 89 ± 10 (*n* = 8), 91 ± 12 (*n* = 17), 68 ± 8 (*n* = 13) and 66 ± 12 (*n* = 13) in animals receiving BP 897 in a range of doses corresponding to minimal to maximal effects of the compound at the D₃ receptor¹⁶, i.e. 0.1, 0.3, 1.0 and 3.0 mg/kg, respectively (F_{4,71} = 3.48, *P* = 0.011, significant effect of BP 897 at 1.0 and 3.0 mg/kg vs. saline, *P* < 0.01). The mixed agonist/antagonist activity of BP 897 thus limited excessive D₃ receptor stimulation, without complete blockade of dopamine function.

BP 897 was administered to dyskinetic MPTP-intoxicated monkeys in combination with levodopa, administered at a fixed and tailored dose that corresponded to the minimal dose producing a full reversal of PD-like symptoms in those monkeys. First step consisted of an open study where doses of BP 897 were tailored for each monkey, i.e. doses were carefully increased until reaching a maximal effect on LID, when administered with a fixed dose of levodopa. These doses were 5.0 (*n* = 1) or 10 mg/kg (*n* = 4) and gave a maximal plasma concentration of BP 897 of 280 ± 110 nM, nearly matching (130 ± 14 nM, *n* = 6) those reached after a BP 897 injection to mice at a dose giving selective effects at the D₃ receptor, as assessed using D₃ receptor gene-targeted mice (Pilla et al., 1999). Thereafter, the trial was performed using a randomized, crossed-over, placebo-controlled design, in which the animals were repeatedly treated (4-12 times by each treatment) and evaluated for LID and PD-like symptoms by experimenters unaware of the treatment received. BP 897 attenuated LID by 66% (calculated from areas under curve, see Fig. 8*a*), but had almost no influence on the therapeutic effect of levodopa, *i.e.* did not reverse the improvement of PD-like symptoms (Fig. 8*b*). Both hyperkinesia (that is not true dyskinesia and thus not rated as one) and choreic/athetoid movements were improved. The few remaining dyskinesia were mainly of dystonic nature. The kinetics of activity counts (Fig. 8*c*) highlight this characteristic pharmacological behavior by showing the ability of BP 897 to undermine the levodopa-induced activity around the dyskinesia threshold, defined from a correlation with clinically-assessed LID (see Methods). The slight effect of BP 897 on improvement of PD-like symptoms by levodopa at t = 150 min (Fig. 8*b*) is not clinically relevant, since on-time duration calculated from activity counts (threshold at 30% of the maximal activity (Grondin et al., 2000)) showed no difference with placebo (Fig. 8*d*). In addition, the monkey receiving repeated administrations of BP 897 (12 times) did not show any obvious signs of decline of the antidyskinetic effect along treatments, nor reappearance of the Parkinson-like symptoms.

It was further investigated whether the characteristic of BP 897 of attenuating LID without ablating the therapeutic response to levodopa was related to the mixed agonist/antagonist activity of this compound. Nafadotride, a D₃ receptor-preferring antagonist (Sautel et al., 1995), was administered to four dyskinetic monkeys in increasing dosage until the threshold dose, i.e. the first active dose on LID was found, which was 0.6 (*n* = 3) or 1.0 mg/kg (*n* = 1) and corresponded to doses producing selective D₃ receptor occupancy in rats. On 3 out of 4 monkeys, nafadotride elicited a reduction of LID similar to that obtained with BP 897 (Fig. 8*a*), which was, however, accompanied by a reappearance of PD-like symptoms (Fig. 8*b*). In one monkey receiving the dose of 1.0 mg/kg, nafadotride attenuated LID (score = 0.5) without affecting the effect of levodopa on PD-like symptoms (score = 0), but a slightly higher dose of nafadotride (1.3 mg/kg) elicited the reappearance of PD-like symptoms in this monkey (the score of LID and PD-like symptoms were 0.5 and 4, respectively). Nafadotride strongly reduced locomotor activity (Fig. 8*c*), most of the time below the on-time threshold, so that the on-time period was markedly reduced (Fig. 8*d*).

Nafadotride has, nevertheless, a limited selectivity towards the D₃ receptor (Sautel et al., 1995 ; Levant et al., 1997) and it could not be excluded that the reappearance of PD-like symptoms produced by nafadotride has resulted from D₂ receptor blockade. Testing this possibility required the use of a highly D₃ receptor-selective antagonist; two of these have been described (Reavill et al., 2000 ; Millan et al., 2000), but were not available to us. In the course of a pharmacochemistry program aiming at the discovery of novel D₃ receptor antagonists, we designed and synthesized ST 198 ((*E*)-*N*-(4-[1,2,3,4-tetrahydroisoquinolin-2-yl]-butyl)-3-phenylacrylamide), compound identified as a novel D₃ receptor-selective ligand (see Example 3).

Initial pharmacokinetic studies in monkeys indicated that ST 198 was rapidly absorbed (maximal plasma concentration reached after 30 min) and eliminated from plasma with a half-life of ~ 30 min. In comparison, BP 897 and nafadotride plasma concentrations reached maximum at 60 min and declined with half-lives of ~ 60 min and ~ 50 min, respectively. ST 198 was administered in escalating dosage to determine the first active dose on LID, as for nafadotride, which was 100 (*n* = 2) or 125 mg/kg (*n* = 2). At these doses, the maximal plasma concentration of ST 198 was 3.6 ± 0.6 µM and identical to that reached in mice for inhibiting dizocilpine-induced hyperlocomotion (3.7 ± 0.2 µM). During the period of time ST 198 was active (30-90 min after administration), LID were attenuated to a similar extent as by nafadotride (Fig. 8*a*), but, again, PD-like symptoms reappeared (Fig. 8*b*) and locomotor activity was ablated below the on-time threshold level (Fig. 8*c*8*c*). Therefore PD-like symptoms deterioration was produced by antagonizing levodopa at the D₃ receptor.

These results indicate that attenuation of LID leaving intact the therapeutic effect of levodopa can be obtained by administration of a D₃ receptor-selective partial agonist, or a D₃ receptor antagonist, but the latter within a very narrow range of doses. Because BP 897 is a partial agonist at the D₃ receptor, but an antagonist at the D₂ receptor (Pilla et al., 1999), the manifestations of the partial agonistic activity of this compound reported here rule out an involvement of the D₂ receptor. In addition, interaction of BP 897 with D₂ receptors is achieved at plasma concentrations that are at least 13 times higher than those required to reduce LID in monkeys, which indicates that the contribution of D₂ receptors in the acute effects of this compound, if any, is limited. The disclosed results therefore suggest that the D₃ receptor participates in the generation of both dyskinetic movements and the therapeutic effects of levodopa. The former hypothesis is fully consistent with the present observation that D₃ receptor is upregulated in the brain of dyskinetic monkeys, particularly in GPi and Pu, two functionally linked brain structures. Since D₃ receptor mRNA is nearly absent from the GPi but expressed in the striatal medium spiny neurons that project upon the GPi neurons, D₃ receptor upregulation reported here would thus occur in a single neuronal population at both the somatodendritic (Pu) and terminal (GPi) levels. Interestingly, the onset of LID is correlated with both over-reduction of GPi firing frequency and abnormal firing pattern that lead, via reduced inhibition of motor thalamic nuclei, to subsequent overactivation of cortical motor areas. Targeting the D₃ receptor in both Pu and GPi may thus account for the dramatic improvement of LID through normalization of functional activity of medium spiny neurons projecting upon the GPi that ultimately results on normalization of GPi neuronal activity.

The intervention of the D₃ receptor in the antiparkinsonian activity of levodopa is quite unexpected, since neither D₃ receptor blockade (Reavill et al., 2000; Millan et al., 2000) nor invalidation (Accili et al., 1996) impairs spontaneous locomotor activity in normal rodents, whereas D₂ receptor blockade or invalidation (Baik et al., 1995) depresses this activity. Thus, it seems that dopamine denervation and subsequent treatment by levodopa have elicited a redistribution of the respective roles of D₂ and D₃ receptors. This is consistent with the D₂ receptor normalization of lesion-induced upregulation *(i.e.* apparent down-regulation vs. lesion-only (Gerfen et al., 1990) and D₃ receptor upregulation (Bordet et al., 1997) following treatments by dopamine agonists. Nevertheless, the D₃ receptor is likely to co-operate with other dopamine receptors subtypes, since BP 897 alone (tested on two dyskinetic monkeys) had no antiparkinson activity and induced rotations in PD-like rats only when associated with a D₁ receptor agonist (Pilla et al., 1999).

Medical management of LID has currently three objectives: (i) to prevent the occurrence of the priming phenomenon, which generates dyskinesia; (ii) to reverse, once primed, the changes occurring in the basal ganglia that induce dyskinesia; or (iii) to avoid the expression of dyskinesia in an already primed brain. Preventing, at least partly, priming for dyskinesia is currently attempted by delaying the introduction of levodopa, and/or reducing the cumulative dose of levodopa, using D₂/D₃ receptor agonists at early stages of the treatment. Reversing dyskinesia priming remains a matter of controversy, since any improvement gained from a drug holiday can be short-lived, and re-introduction of levodopa can re-prime the patient. Reducing the dose of levodopa and using other drugs as adjuncts has proved inconsistency of benefit.

The disclosed results here provide a novel therapeutic approach based on the use, in combination with levodopa, of a D₃ receptor partial agonist, which presently showed clear advantage over D₃ receptor antagonists, to avoid the expression of dyskinesia in already primed patients, i.e. the today largest population of PD patients.

### REFERENCES

Accili, D., Fishburn, C.S., Drago, J., Steiner, H., Lachowicz, J.E., Park, B.-H., Gauda, E.B., Lee, E.J., Cool, M.H., Sibley, D.R., Gerfen, C.R., Westphal, H., Fuchs, S., (1996) A targeted mutation of the D₃ receptor gene is associated with hyperactivity in mice. Proceeding of the National Academy of Sciences of the USA 93, 1945-1949.

Andiné, P., Widermark, N., Axelsson, R., Nyberg, G., Olofsson, U., Martensson, E., Sandberg, M., (1999) Characterization of MK-801-induced behavior as a putative rat model of psychosis. The Journal of Pharmacology and Experimental Therapeutics 290, 1393-1408.

Baik, J.H., *et al.* (1995) Parkinsonian-like locomotor impairment in mice lacking dopamine D₂ receptors. *Nature* **377,** 424-428.

Bézard, E., Brotchie, J.M. & Gross, C.E. (2001) Pathophysiology of levodopa-induced dyskinesia: potential for new therapies. *Nat. Rev. Neurosci.* **2,** 577-587.

Bordet, R., Ridray, S., Carboni, S., Diaz, J., Sokoloff, P., Schwartz, J.-C., (1997) Induction of dopamine D₃ receptor expression as a mechanism of behavioral sensitization to levodopa. Proceeding of the National Academy of Sciences of the USA 94, 3363-3367.

Burns, R.S., *et al.* (1983) A primate model of parkinsonism: selective destruction of dopaminergic neurons in the pars compacta of the substantia nigra by N-methyl-4-phenyl-1,2,3,6-tetrahydropyridine. *Proc. Natl. Acad. Sci. USA* **80**, 4546-4550.

Carlsson, A., (1988) The current status of the dopamine hypothesis of schizophrenia. Neuropsychopharmacology 1, 179-186.

Carlsson, M., Carlsson, A., (1989) The NMDA antagonist MK-801 causes marked locomotor stimulation in monoamine-depleted mice. Journal of Neural Transmission 75, 221-226.

Corbett R, Hartman H, Kerman LL, Woods AT, Strupczewski JT, Helsley GC, Conway PC, Dunn RW (1993) Effects of atypical antipsychotric agents on social behavior in rodents. *Pharmacol Biochem Behav* **45**:9-17.

Cosgrove, J., Newell, T.G., (1991) Recovery of neuropsychological function during reduction in use of phencyclidine. Journal of Clinical Psychology 47, 159-169.

Cotzias, G.C., Papavasiliou, P.S. & Gellene, R. (1969) Modification of Parkinsoninsm-chronic treatment with L-dopa. *New Eng. J. Med.* **280,** 337-345.

Diaz, J., Pilon, C., Le Foll, B., Gros, C., Triller, A., Schwartz, J.-C., Sokoloff, P., (2000) Dopamine D₃ receptors expressed by all mesencephalic dopamine neurons. Journal of Neuroscience 20, 8677-8684.

Dixon AK, Huber C, Lowe DA (1994) Clozapine promotes approach-oriented behavior in male mice. *J Clin Psychiatry* **55** Suppl B:4-7.

Dubertret, C., Gorwood, P., Ades, J., Feingold, J., Schwartz, J.-C., Sokoloff, P., (1998) A meta-analysis of DRD₃ gene and schizophrenia: ethnic heterogeneity and significant association in caucasians. American Journal of Medical Genetic [Neuropsychiatric Genetics] 81, 318-322.

Gerfen, C.R., *et al.* (1990) D₁ and D₂ dopamine receptor-regulated gene expression of striatonigral and striatopallidal neurons. *Science* **250,** 1429-1432.

Geter-Douglas and, Witkin. (1997) Dizocilpine-like discriminative stimulus effects of competitive NMDA receptor antagonists in mice. Psychopharmocology, 133 : 43-50.

Grenhoff, J., Nisell, M., Ferre, S., Aston-jones, G., Svensson, T.H., (1993) Noradrenergic modulation of midbrain dopamine cell firing elicited by stimulation of the locus coeruleus in the rat. Journal of Neural Transmission 93, 11-25.

Grondin, R., Tahar, A.H., Doan, V.D., Ladure, P. & Bedard, P.J. (2000) Noradrenoceptor antagonism with idazoxan improves L-dopa-induced dyskinesias in MPTP monkeys. *Naunyn-Schmiedebergs Arch. Pharmacol.* **361,** 181-186.

Gurevich, E.V., Bordelon, Y., Shapiro, R.M., Arnold, S.E., Gur, R.E., Joyce, J.N., (1997) Mesolimbic dopamine D₃ receptors and use of antipsychotics in patients with schizophrenia. Archives of General Psychiatry 54, 225-232.

Imbert, C., Bezard, E., Guitraud, S., Boraud, T. & Gross, C.E. (2000) Comparison of eight clinical rating scales used for the assessment of MPTP-induced parkinsonism in the Macaque monkey. *J. Neurosci. Meth.* **96,** 71-76.

Irifune et al. (1995) Involvement of N-methyl-D-aspartate (NMDA) receptors in noncompetitive NMDA receptor antagonist-induced hyperlocomotion in mice. Pharmacol. Biochem. Behav., 51:291-296

Ital, T., Keskiner, A., Kiremitci, N., Holde, J.M.C., (1967) Effect of phencyclidine in chronic schizophrenics. Canadian Psychiatric Association Journal 12, 209-212.

Janowsky, D.S., Davis, J.M., (1976) Methylphenidate, dextroamphetamine, and levamfetamine. Effects on schizophrenic symptoms. Archives of General Psychiatry 33, 304-308.

Javitt, D.C., Zukin, S.R., (1991) Recent advance in the phencyclidine model of schizophrenia. American Journal of Psychiatry 148, 1301-1308.

Jentsch JD, Redmond DEJ, Elsworth JD, Taylor JR, Youngren KD, Roth RH (1997) Enduring cognitive deficits and cortical dysfunction in monkeys after long-term administration of phencyclidine. *Science* **277**:953-955.

Jentsch, J., Roth, R., (1999) The neuropsychopharmacology of phencyclidine: from NMDA receptor hypofunction to the dopamine hypothesis of schizophrenia. Neuropsychopharmacology 20, 201-225.

Krystal JH, Karper LP, Seibyl JP, Freeman GK, Delaney R, Bremne JD, Heninger GR, Bowers MB Jr, Charney, DS. Subanesthetic effects of the non competitive NMDA antagonist ketamine, in humans. Psychomimitic, perceptual, cognitive and neuroendocrine responses. Arch Gen Psychiatry, 1994, 51: 199-214.

Laruelle M (2000) The role of endogenous sensitization in the pathology of schizophrenia: implications from recent brain imaging studies. *Brain Res Rev* **31**:371-384.

Lawler CP, Prioleau C, Lexis MM, Mak C, Jinag D, Schetz JA, Gonzales AM, Sibley DR and Mailman RB. Interactions of the novel antipsychotic aripripazole (OPC-14597) with dopamine and serotonin receptor subtypes. Neuropsychopharmacology, 1999, 20:612-627.

Le Foll, B., Francès, H., Diaz, J., Schwartz, J.-C., Sokoloff, P., (2002) Role of the dopamine D₃ receptor in reactivity to cocaine-associated cues in mice. European Journal of Neuroscience 15, 2016-2026.

Levant, B., Vansell, N.R., (1997) In vivo occupancy of D₂ dopamine receptors by nafadotride. Neuropsychopharmacology 17, 67-71.

Lévesque, D., *et al*. (1995) A paradoxical regulation of the dopamine D₃ receptor expression suggests the involvement of an anterograde factor from dopamine neurons. *Proc. Natl. Acad. Sci. USA* **92,** 1719-1723.

Luby, E.D., Cohen, B.D., Rosenbaum, G., Gottlieb, J.S., Kelly, R., (1959) Study of a new schizohprenic-like drug: Sernyl. Archives of Neurology and Psychiatry 81, 363-369.

Mathé, J.M., Nomikos, G.G., Hildebrand, B.E., Hertel, P., Svensson, T.H., (1996) Prazosin inhibits MK-801-induced hyperlocomotion and dopamine release in the nucleus accumbens. European Journal of Pharmacology 309, 1-11.

Meller, E., Helmer-Matyjek, E., Bohmaker, K., Adler, C.H., Friedhoff, A.J., Golstein, M., (1986) Receptor reserve at striatal dopamine autoreceptors : implications for selectivity of dopamine agonists. European Journal of Pharmacology 123, 311-314.

Meltzer, H.Y., (1994) An overview of the mechanism of action of clozapine. Journal of Clinical Psychiatry 55, 47-52.

Millan, M.J., Gobert, A., Newman-Tancredi, A., Lejeune, F., Cussac, D., Rivet, J.-M., Audinot, V., Dubuffet, T., Lavielle, G., (2000) S33084, a novel potent, selective, and competitive antagonist at dopamine D₃-receptors: I receptorial, electrophysiological and neurochemical profile compared with GR218,231 and L741,626. The Journal of Pharmacology and Experimental Therapeutics 293, 1048-1062.

Murray, T.F., Horita, A., (1979) Phencyclidine-induced stereotyped behavior in rat: dose-response effects and antagonism by neuroleptics. Life Sciences 24, 2217-2225.

Ninan, I., Kulkarni, S.K., (1999) Preferential inhibition of dizocilpine-induced hyperlocomotion by olanzapine. European Journal of Pharmacology 368, 1-7.

Ögren, S.O., Goldstein, M., (1994) Phencyclidine- and dizocilpine- induced hyperlocomotion are differentially mediated. Neuropsychopharmacology 11, 167-177.

O'Neill, M.F., Heron-Maxwell, C.L., Shaw, G., (1999) 5-HT₂ receptor antagonism reduces hyperactivity induced by amphetamine, cocaïne, and MK-801 but not D₁ agonist C-APB. Pharmacology Biochemistry and Behavior 63, 237-243.

Pearce, R.K., Jackson, M., Smith, L., Jenner, P. & Marsden, C.D. (1995) Chronic L-DOPA administration induces dyskinesias in the 1-methyl-4- phenyl-1,2,3,6-tetrahydropyridine-treated common marmoset (Callithrix Jacchus). *Mov. Disord.* **10**, 731-740.

Perachon, S., Betancur, C., Pilon, C., Rostene, W., Schwartz, J.-C., Sokoloff, P., (2000) Role of dopamine D₃ receptors in thermoregulation: a reappraisal. Neuroreport 11, 221-225.

Pilla, M., Perachon, S., Sautel, F., Garrido, F., Mann, A., Wermuth, C.G., Schwartz, J.-C., Everitt, B.J., Sokoloff, P., (1999) Selective inhibition of cocaine-seeking behaviour by a partial dopamine D₃ receptor agonist. Nature 400, 371-375.

Pilon, C., *et al.* (1994) Functional coupling of the human dopamine D₃ receptor in a transfected NG 108-15 neuroblastoma-glioma hybrid cell line. *Eur. J. Pharmacol. [Mol. Pharmacol. Sect*.] **268,** 129-139.

Prisco, S., Esposito, E., (1995) Differential effects of acute and chronic fluoxetine administration on the spontaneous activity of dopaminergic neurones in the ventral tegmental area. British Journal of Pharmacology 116, 1923-1931.

Reavill, C., Taylor, S.G., Wood, M.D., Ashmeade, T., Austin, N.E., Avenell, K.Y., Boyfield, I., Branch, C.L., Cilia, J., Coldwell, M.C., Hadley, M.S., Hunter, A.J., Jeffrey, P., Jewitt, F., Johnson, C.N., Jones, D.N.C., Medhurst, A.D., Middlemiss, D.N., Nash, D.J., Riley, G.J., Routledge, C., Stemp, G., Thewlis, K.M., Trail, B., Vong, A.K.K., Hagan, J.J., (2000) Pharmacological actions of a novel high-affinity, and selective human dopamine D₃ receptor antagonist, SB-277011-A. The Journal of Pharmacology and Experimental Therapeutics 294, 1154-1165.

Sautel, F., Griffon, N., Sokoloff, P., Schwartz, J.-C., Launay, C., Simon, P., Costentin, J., Schoenfelder, A., Garrido, F., Mann, A., Wermuth, C.G., (1995) Nafadotride, a potent preferential dopamine D₃ receptor antagonist, activates locomotion in rodents. The Journal of Pharmacology and Experimental Therapeutics 275, 1239-1246.

Schaefer, G.J., Michael, R.P., (1984) Drug interactions on spontaneous locomotor activity in rats. Neuroleptics and amphetamine-induced hyperactivity. Neuropharmacology 23, 909-914.

Schotte, A., Janssen, P.F., Gommeren, W., Luyten, W.H., Leysen, J.E., (1992) Autoradiographic evidence for the occlusion of rat brain dopamine D₃ receptors *in vivo.* European Journal of Pharmacology 218, 373-375.

Schwartz, J.-C., Diaz, J., Pilon, C., Sokoloff, P., (2000) Possible implications of the dopamine D₃ receptor in schizophrenia and in antipsychotic drug actions. Brain Research Reviews 31, 277-287.

Snyder, S.H., (1988) Psychogenic drugs as models of schizophrenia. Neuropsychopharmacology 1, 197-199.

Sokoloff et al. (1990) Molecular cloning and characterization of a novel dopamine receptor (D3) as a target for neuroleptics. Nature, 13;347(6289):146-51

Suzuki, M., Hurd, Y.I., Sokoloff, P., Schwartz, J.C., & Sedvall, G. (1998) D₃ dopamine receptor mRNA is widely expressed in the human brain. *Brain Res*.**779**:58-74.

Svensson, T.H., (2000) Dysfunctional brain dopamine systems induced by psychotomimetic NMDA-receptor antagonists and the effects of antipsychotic drug. Brain Research Reviews 31, 320-329.

Weber, B., Schlicker, E., Sokoloff, P. & Stark, H. (2001) Identification of the dopamine autoreceptor in the guinea-pig retina as D₂ receptor using novel subtypeselective antagonists. *Br. J. Pharmacol.* **133,** 1243-1248.

Wong, E.H.F., Kemp, J.A., Priestley, T., Knight, A.R., Woodruff, G.N., Iversen, L.L., (1986) The anticonvulsivant MK-801 is a potent N-methyl-D-aspartate antagonist. Proceeding of the National Academy of Sciences of the USA 83, 7104-7108.

Xu, M., Koeltzow, T.E., Cooper, D.C., Tonegawa, S., White, F.J., (1999) Dopamine D₃ receptor mutant and wild-type mice exhibit identical responses to putative D₃ receptor-selective agonists and antagonists. Synapse 31, 210-215.

Zapata, A., Witkins, J.M., Shippenberg, T.S., (2001) Selective D₃ receptor agonist effects of (+)-PD 128907 on dialysate dopamine at low doses. Neuropharmacology 41, 351-359.

## Claims

1. A method for identifying a dopamine D₃ receptor ligand having anti-dopamine D₃ receptor activity *in vivo,* said method comprising a) administering to a mammal (i) a candidate molecule and (ii) a NMDA glutamate receptor antagonist in a amount suitable for inducing dopamine D3 receptor-dependent psychotic behavior in said mammal, and
b) determining the capacity of the candidate molecule to prevent or inhibit the psychotic behavior induced in the mammal, wherein a reduced of the psychotic behavior or non-appearance thereof is indicative of a dopamine D₃ receptor ligand having anti-dopamine D₃ receptor activity *in vivo.*

2. The method according to claim 1, wherein the candidate molecule is administered prior to the NMDA glutamate receptor antagonist, and the psychotic behavior of the mammal is assessed before and after each administration, and compared with the behavior of a control mammal to which a control vehicle has been administered in lieu of the candidate molecule;
wherein a reduction of psychotic behavior, or non-appearance thereof, with comparison to the behavior of the control mammal is indicative of a dopamine D₃ receptor ligand having anti-dopamine D₃ receptor activity *in vivo.*

3. The method according to claim 1, wherein the candidate molecule is administered after the NMDA glutamate receptor antagonist, and the psychotic behavior of the mammal is assessed before and after each administration, and compared with the behavior of a control mammal to which a control vehicle has been administered in lieu of the candidate molecule;
wherein a reduced psychotic behavior with comparison to the behavior of the control mammal is indicative of a dopamine D₃ receptor ligand having anti-dopamine D₃ receptor activity *in vivo.*

4. The method according to claim 3, wherein said NMDA glutamate receptor antagonist is administered continuously to said mammal for 1 to 10 days, prior to administrating the candidate molecule.

5. The method according to any of claims 1 to 4, wherein said *in vivo* anti-dopamine D₃ receptor activity is an anti-psychotic activity.

6. The method according to any of claims 1 to 5, wherein said NMDA glutamate receptor antagonist is selected from the group consisting of phencyclidine, TCP, ketamine, (+)-MK-801, dextrorphan, SKF 10,047, CGS 19755, NCP 17742, and (±)-CPP.

7. The method according to any of claims 1 to 6, wherein said psychotic behavior is a psychotic-like behavior that includes spontaneous locomotor hyperactivity, enhanced responses to psychostimulants, deficit in spatial and working memory, or deficit in social interaction.

8. The method according to any of claims 1 to 6, wherein said psychotic behavior is a substance-induced psychotic behavior that includes alterations of perception, impaired performance on tests of vigilance, attention and verbal fluency, impaired performance on the Wisconsin Card Sorting Test, symptoms similar to dissociative states, alteration of preattentive auditory event-related potential, impaired recall and recognition memories, alterations of scores on the Brief Psychiatric Rating Scale, Scale for the Assessment of positive Symptoms and Scale for Assessment of Negative Symptoms.

9. The method according to any of claims 1 to 8, wherein said mammal is selected from the group consisting of a human, a mouse, a rat, a monkey, a dog, and a cat.

10. The use of a dopamine D₃ receptor ligand having anti-D₃ receptor activity *in vivo* for the manufacture of a medicament intended for the treatment of a substance-induced psychotic disorder in a patient in need thereof.

11. The use according to claim 10, wherein said dopamine D₃ receptor ligand is a partial agonist or an antagonist of the D₃ receptor having anti-psychotic activity *in vivo.*

12. The use according to claim 10 or 11, wherein said substance-induced psychotic disorder is associated with the administration of a NMDA receptor antagonist.

13. The use according to any of claims 10 to 12, wherein said substance-induced psychotic disorder is selected from the group consisting of a psychotic disorder associated with administration of an anesthetic, or a medication for the treatment of Alzheimer's disease, vascular dementia or Parkinson's disease, and with abuse of a psychoactive drug.

14. Use of a partial agonist of the dopamine D₃ receptor for the manufacture of a medicament intended for the treatment of a dyskinesia associated with dopamine agonist therapy.

15. The use according to claim 14, wherein said dyskinesia is associated with a dopamine agonist therapy used in the treatment of Parkinson's disease.

16. The use according to claim 14 or 15, wherein said partial agonist of the dopamine D3 receptor is selected from the group consisting of BP 897 and aripiprazole.
